Europäisches Patentamt

European Patent Office

Office européen des brevets

(19)

(11) **EP 1 473 148 A1**

(12) **EUROPÄISCHE PATENTANMELDUNG**

(43) Veröffentlichungstag:
03.11.2004  Patentblatt 2004/45

(51) Int Cl.⁷: **B32B 3/14**, B32B 5/26,
A61F 13/15

(21) Anmeldenummer: 03023165.8

(22) Anmeldetag: **13.10.2003**

(84) Benannte Vertragsstaaten:
**AT BE BG CH CY CZ DE DK EE ES FI FR GB GR
HU IE IT LI LU MC NL PT RO SE SI SK TR**
Benannte Erstreckungsstaaten:
**AL LT LV MK**

(30) Priorität: **30.04.2003  DE 10319754**

(71) Anmelder: **Carl Freudenberg KG**
**69469 Weinheim (DE)**

(72) Erfinder:
• **Groitzsch, Dieter, Dr.**
  **69493 Hirschberg (DE)**
• **Grimm, Hansjörg**
  **69469 Weinheim (DE)**
• **Hirn, Nikolaus**
  **69493 Hirschberg (DE)**
• **Knehr, Eric**
  **67688 Rodenbach (DE)**

(54) **Elastischer Verbundstoff, Verfahren zu dessen Herstellung und dessen Verwendung**

(57) Beschrieben wird ein elastischer Verbundstoff umfassend mindestens einen Vliesstoff, mindestens ein weiteres Flächengebilde und zwischen diesen angeordnet eine parallel verlaufende Schar von elastischen Fäden. Der Vliesstoff ist dabei mit dem weiteren Flächengebilde in Form eines vorbestimmten Musters thermisch verschweißt und die elastischen Fäden sind in gespanntem Zustand an ausgewählten Stellen in die Verschweißungen zwischen dem Vliesstoff und dem weiteren Flächengebilde eingebettet.

Der Verbundstoff lässt sich zur Fertigung von Hygieneartikeln, insbesondere von Windeln einschließlich von Windelhöschen, einsetzen.

**Beschreibung**

Technisches Gebiet

Die vorliegende Erfindung betrifft einen elastischen Verbundstoff, der sich insbesondere zur Herstellung von Hygieneprodukten eignet, ein Verfahren zu dessen Herstellung sowie dessen Verwendung zur Herstellung von Hygieneprodukten, wie Einmalwindeln.

Elastische Komponenten werden schon seit Jahren in Einmalwindeln für Erwachsene und Kinder oder in Windelhöschen für unterschiedliche Zwecke eingesetzt. Die Beinabschlussmanschette derartiger Windel enthalten einzelne grobe elastische Fäden zwischen zwei Vliesstofflagen eingeklebt, um eine optimale Anpassung an die Körperform zu gewährleisten und dadurch eine Leckage von Körperflüssigkeiten zu verhindern. Die Gürtelregion einer Windel kann aus elastischen Verbundstoffen bestehen, ebenfalls zum Zweck einer optimalen Anpassung an den Körper.

Aus dem gleichen Grund kann der Verhakungsteil eines mechanischen Verschlusssystems einer Windel auf einem elastischen Träger fixiert sein. Sowohl bei Einmalwindeln als auch bei Windelhöschen werden wenige einzelne elastische Fäden mit sehr hohem Titer von einigen hundert bis einigen tausend dtex im Laufe des Windelfertigungsprozessen inkorporiert. Dabei werden die elastischen Fäden in gedehntem Zustand zwischen zwei Vliesstofflagen geklebt. Der Kleber ist im Normalfall ein Haftkleber (PSA-Kleber, wobei PSA für Pressure Sensitive Adhesive steht), der zumindest auf eine der beiden Vliesstofflagen ganz oder teilflächig aufgetragen wird. Der Abstand zwischen den einzelnen, benachbarten und parallel zueinander ausgerichteten elastischen Fäden ist relativ groß und liegt im Bereich von einigen mm bis zu einem cm. Nach Entspannung der gedehnten elastischen Fäden entsteht durch die groben Fältelungen bzw. Aufwerfungen der unelastischen Vliesstoffschichten eine sehr voluminöse, grobe und optisch wenig ansprechende Struktur. Außerdem vermittelt der direkte Körperkontakt einer solchen Grobfältelung eine ungenehmes Tragegefühl, das im Extremfall mit Abdruckstellen auf der menschlichen Haut verbunden ist und gegebenenfalls die Entstehung von Hautirritationen (Hautrötung) fördert.

Stand der Technik

Elastische Gewirke, welche die oben genannten Nachteile nicht aufweisen, haben sich bislang nicht durchsetzen können, da diese zu teuer und zu hochwertig sind, um in einem Wegwerfartikel eingesetzt werden zu können. Das Einwirken von relativ feintitrigen, gedehnten elastischen Fäden (Rascheltechnik; Maliwatt) im Bereich von 44 bis ca. 200 dtex führt zu optisch sehr ansprechenden, elastischen textilen Flächengebilden, insbesondere dann, wenn sowohl die Einstichzahl (Maschenzahl in Kettrichtung = Maschinenlaufrichtung) als auch die sogenannte Teilung (also die Anzahl der Fäden bzw. Garne pro Längeneinheit quer zur Maschinenlaufrichtung) hoch ist. Das Problem solcher nähgewirkten (stitch bonded) elastischen Produkte liegt jedoch darin, dass die elastischen Fäden in dem Flächengebilde nicht fixiert sind und sich deswegen nach dem Schneiden oder Ausstanzen bei mechanischer Belastung (Spannen und Entspannen) aus dem Verbund lösen. Zur Fertigung eines elastischen Windelhöschens aus einem Vorder- und Hinterteil müsste die Fäden vor dem Ausstanzen an den Rändern mit dem Vliesstoff verschweißt oder geklebt werden. Dies würde die Herstellung einer Windel bzw. eines elastischen Höschens zu sehr erschweren.

Bekanntermaßen lassen sich die Beträge an maximaler Dehnung durch die Höhe der Verdehnung der elastischen Fäden vor dem Kleben in den oder die beiden unelastischen Träger beliebig einstellen. In der WO-A-00/20202 wird die Herstellung eines Verbundstoffes aus zwei Lagen Vliesstoff (Polyester-Spunlaced bzw. Polypropylen-Spinnvliesstoffe) und dazwischen eingelegten Spandex-Elastanfäden beschrieben. Die Bindung der Spandex-Elastanfäden erfolgt durch zusätzliches Auftragen von Schmelzkleber nach dem sogenannten Meltblown-Prinzip, um die Haftung der elastischen Fäden an den Vliesstoffschichten zu gewährleisten.

In der US-A-6,179,946 werden Verbunde beschrieben, bei denen in Querrichtung abgelegte Spandex-Elastanfäden mit Hilfe von aufgesprühtem Schmelzkleber an die beiden Vliesstofflagen gebunden sind.

In der US-A-6,086,571 werden beide Teile eines elastischen Gürtels einer Windel beschrieben, an deren Ende ein Verhakungsteil bzw. ein Einhakungsteil eines mechanischen Verschlusssystems angebracht ist. Auch hier wird erwähnt, dass die gedehnten elastischen Fäden mit Hilfe von Adhäsion oder Schmelzkleber an die Polyproyplen-Spinnvliesstoffe angeklebt sind.

In der EP-A-677,284 wird ein seitlicher Auslaufschutz einer Windel beschrieben, in der ein elastomerer Faden zwischen zwei hydrophoben Polypropylen-Spinnvlieslagen auf spezielle Weise eingebunden ist. Anstelle der Faltung eines Spinnvliesstoffes um den eingelegten elastomeren Faden und Hotmelt-Verklebung desselben mit den beiden Spinnvlieslagen zum Zwecke der Einkapselung des Fadens werden zwei separate Vliesstofflagen an deren Ende mit einem ersten Prägemuster miteinander thermisch verschweißt, so dass der elastische Faden nicht seitlich entweichen kann. Die unterbrochenen Prägemuster können ein- oder beidseitig des elastischen Fadens angeordnet sein. Auf den gedehnten elastischen Faden zwischen den beiden Vliesstofflagen wird ein zweites intermittierendes Prägemuster aufgesetzt, um den Faden zwischen den Schweißlinien der beiden Vliesstofflagen gefangen zu halten. Damit konnte der zusätzliche Auftrag von Schmelzkleber eingespart und eine Versteifung der Ware verhindert, d.h. eine bessere Weichheit erzielt werden.

Die aus dem beschriebenen Stand der Technik resultierenden Produkte mit zusätzlichem Auftrag an Schmelzkleber führen zu einer Versteifung des Flächengebildes und zu einem hohen Rohstoffverbrauch zur Bindung des gedehnten elastischen Fadens, zu einer Verkomplizierung des Laminierverfahrens und zu relativ hohen Kosten.

Ein schmelzkleberfreies Einbringen eines elastischen Fadens ist gemäß EP-A-677,284 zwar prinzipiell gelungen, jedoch beschränkt sich diese Methode auf den seitlichen Auslaufschutz einer Windel mit einem einzigen oder lediglich zwei eingelagerten Fäden.

Darstellung der Erfindung

Aufgabe der vorliegenden Erfindung ist es, die Nachteile des diesbezüglich genannten Standes der Technik zu beheben und elastische Verbundstoffe bereitzustellen, die in den unterschiedlichsten Positionen eines Hygieneartikels, wie einer Windel oder einem Windelhöschen, eingesetzt werden können.

Eine weitere Aufgabe der Erfindung ist auch die Bereitstellung von elastischen Verbundstoffen, die in Weichheit und Textilität denen einer nähgewirkten (geraschelten) Ware mit Vliesstoff als Träger sehr nahe zu kommen bzw. die bekannten schmelzkleber- oder haftkleber-gebunden elastischen Vliesstoff-Faden-Laminate übertreffen.

Die erfindungsgemäßen elastischen Flächengebilde weisen eine gegenüber den vorbekannten Vliesstoff-Faden-Laminaten verbesserte Textilität und optische Gestaltung auf, die es ihnen erlaubt, nach dem Einbau als Komponenten in eine Windel und/oder Windelhöschen, deren Passform und Anpassungsfähigkeit an körperliche Formen und damit auch den Tragekomfort und zu verbessern und das Risiko einer Leckage von Körperflüssigkeiten im Vergleich zu bekannten Produkten zu verringern.

Eine weitere Aufgabe der Erfindung ist in der Bereitstellung von elastischen Verbundstoffen zu sehen, bei deren Aufbau und Herstellungsmethode völlig auf die Verwendung eines zusätzlichen Klebers für die Bindung der elastischen Fäden auf den Vliesstofflagen verzichtet werden kann, ohne dass dadurch die Intensität der Einbettung oder Verankerung der elastischen Fäden in dem Verbundstoff verschlechtert wird.

Die vorliegende Erfindung betrifft einen Verbundstoff umfassend mindestens einen Vliesstoff, mindestens ein weiteres Flächengebilde und zwischen diesen angeordnet eine parallel verlaufende Schar von elastischen Fäden, dadurch gekennzeichnet, dass der Vliesstoff mit dem weiteren Flächengebilde in Form eines vorbestimmten Musters thermisch verschweißt ist und dass die elastischen Fäden in gespanntem Zustand an ausgewählten Stellen in die Verschweißungen zwischen dem Vliesstoff und dem weiteren Flächengebilde eingebettet sind.

Durch die Einbettung der elastischen Fäden zwischen Vliesstoff und weiterem Flächengebilde an Stellen der thermischen Verschweißung werden diese in gespanntem Zustand rutsch- und zerstörungsfrei in den Verbundstoff eingelagert. Dieses kann ohne die Anwesenheit eines zusätzlichen Haft- oder Bindemittels auf den elastischen Fäden und/oder zwischen dem Vliesstoff und dem weiteren Flächengebilde erfolgen.

Der erfindungsgemäße Verbundstoff weist mindestens eine Lage Vliesstoff und mindestens eine Lage eines weiteren Flächengebildes auf. Dieses kann ebenfalls ein Vliesstoff sein oder eine Folie. Der Vliesstoff und/oder das weitere Flächengebilde können schrumpfarm sein oder derart ausgestaltet sein, dass es unter Einwirkung von feuchter und/oder trockener Hitze zum Schrumpfen bzw. zur Verkleinerung seiner Fläche neigt. Der Vliesstoff und/oder das weitere Flächengebilde können selbst elastisch oder starr sein. Der Vliesstoff ist vorzugsweise nicht elastisch.

Die erfindungsgemäß zum Einsatz kommenden Vliesstoffe können aus beliebigen Fasertypen der verschiedensten Titerbereiche bestehen, beispielsweise der Titer von 0,5 bis 10 dtex, vorzugsweise von 0,8 bis 6,7 dtex, insbesondere von 1,3 bis 3,3 dtex. Neben Homofilfasern können auch Heterofilfasern, beispielsweise Bikomponentenfasern, in gekräuselter oder ungekräuselter Form oder Gemische verschiedenster Fasertypen eingesetzt werden.

Die Fasern sind vorzugsweise weiß pigmentiert. Zur Farbgebung kann der Schmelzmasse des faserbildenden Polymers Farbstoff zugesetzt werden.

Zur Erreichung einer besonderes weichen Ware werden Vliesstoffe aus zweidimensional oder dreidimensional gekräuselten Bikomponenten-Fasern bevorzugt.

Die erfindungsgemäß eingesetzten Vliesstoffe können mit unterschiedlichen Ablegemethoden gebildet worden sein. Als Schichten können nassgelegte Vliesstoffe, kardierte Stapelfaservliesstoffe, Endlosfilamentvliesstoffe, Meltblown-Vliesstoffe, Spunbond-MeltblownSpunbond-Vliesstoffe (SMS) und, Spunbond-Meltblown-Vliesstoffe (SM) eingesetzt werden. Im letzteren Fall ist es vorteilhaft, wenn die Meltbbown-Schicht im Verbundstoff nach innen, d.h. die elastischen Fäden berührend, gerichtet ist.

Neben Spinnvliesstoffen werden vorzugsweise Stapelfaservliesstoffe, ganz besonders bevorzugt ungebundene Vliesstoffe (Flore) eingesetzt.

Als Vliesstoffe können auch lose Faserflore eingesetzt werden, die nach bekannten Vlieslegetechniken gebildet worden sind. Die Fasern können isotrop oder in einer Vorzugsrichtung, d.h. anisotrop abgelegt worden sein. Der Faserflor kann vor der Laminierung mit mindest einer Faservliesstoffschicht mit bekannten Methoden vorverfestigt sein. Der Faserflor kann aus gleichen oder unterschiedlichen Titern derselben Faser bestehen. Die den Vliesstoff oder Flor aufbauenden Fasern können aus unterschiedlichsten Fasern aufgebaut sein, beispielsweise aus Homofilfasern, aber auch aus 100 % Bikomponenten-Fasern oder einem Verschnitt aus Bikomponenten-Fasern und Homofilfasern, mit der Einschränkung, dass das höher schmelzende Polymer im Falle einer Kern-Mantel-Faser als Kern-Komponente eingesetzt wird.

Beovrzugte Bikomponentenfasem sind solche aus den Polymerkombinationen Polypropylen/Co-Polypropylen und Polypropylen/Polyethylen, wobei solche Verschnitte aus Bikomponentenfasern und Homofil-Fasern ganz besonders bevorzugt werden, bei denen die Homofilfaser identisch ist mit der niedriger schmelzenden Komponente der Bikomponentenfaser. Ein Beispiel hierfür ist ein Verschnitt aus der Bikomponentenfaser Polypropylen/Polyethylen mit der Homofilfaser Polyethylen.

Die Flor- oder Vliesstoffschicht kann mit bekannten Methoden perforiert worden sein oder eine netzartige Struktur aufweisen.

Solche Methoden der Perforation oder Strukturbildung werden bevorzugt, die auf dem Prinzip eines musterartiges Zurseiteschiebens der Fasern beruhen. Solche nicht materialzerstörenden Verfahren sind in EP-A-919,212 und EP-A-789,793 beschrieben.

Es können auch die unten bei der Folie beschriebenen Perforationsverfahren angewendet werden.

Die erfindungsgemäß zum Einsatz kommenden Vliesstoffe sind unter den Herstellungsbedingungen des Verbundstoffes vorzugsweise schrumpffrei.

Typischerweise weisen die zum Einsatz kommenden Vliesstoffe bzw. deren ungebundene Vorstufen (Flore) Flächengewichte von 6 bis 70 g/m$^2$ auf.

Besonders bevorzugt kommen Vliesstoffe mit geringen Flächengewichten von 6 bis 40 g/m$^2$ zum Einsatz. Aus diesen Vliesstoffen lassen sich besonders leichtgewichtige und gleichzeitig hochsaugfähige Verbunde herstellen.

Besonders bevorzugt kommen Endlosfilamentvliesstoffe aus Homofil-Fasern oder Bikomponenten-Fasern mit olefinischer Polymerzusammensetzung zum Einsatz.

Beispiele hiefür sind solche aus Polypropylen, Polyethylen und olefinischen Copolymeren, die beispielsweise entweder mithilfe von Ziegler-Natta-oder Metallocen-Katalysatoren hergestellt worden sind.

Das weitere Flächengebilde kann beliebiger Natur sein. Dabei kann es sich um ein Faserflächengebilde handeln, beispielsweise um Gewebe, Gewirke, Netz, Gitter und Gelege oder insbesondere um einen Vliesstoff handeln oder es kann sich um eine Folie handeln, sofern dieses weitere Flächengebilde mit dem ersten Vliesstoff verschweißbar ist.

Das weitere Flächengebilde kann aus verstreckten, linear ausgerichteten und parallel zueinander orientierten Fäden oder Garnen bestehen. Die verstreckten bzw. gereckten Fäden oder Monofilamente können durch andere in einem Winkel zu den ersteren ausgerichteten verstreckten oder nicht verstreckten oder weniger verstreckten Fäden/ Monofilamente bzw. Garnen bestehen. Die sich überkreuzenden Fasern, Fäden oder Monofilamente können durch Eigenbindung, beispielsweise durch mechanische Bindung oder durch Verschweißen an den Kreuzungspunkten, an die anderen gebunden sein. Die Bindung kann aber auch durch Bindemittel, wie wässrige Dispersionen erfolgt sein.

Das weitere Flächengebilde des Verbundstoffes kann aus einer uniaxial- oder biaxial gereckten Folie bestehen. Die Folie kann nach den bekannten Herstellverfahren erzeugt worden sein, beispielsweise nach dem Blasverfahren, d.h. in Schlauchform gereckt worden sein. Sie kann aber auch durch Extrusion durch eine Breitschlitzdüse geformt und durch mechanisches Recken in Maschinenlaufrichtung gelängt worden sein oder quer zur Maschinenlaufrichtung durch einen Spannrahmen oder durch Passieren eines ineinandergreifenden Walzenpaares mit Rillierung in Maschinenlaufrichtung gereckt worden sein.

Das übliche Reckverhältnis der Folie liegt bei bis zu 5 : 1 in einer oder beiden Reckrichtungen. Unter Reckverhältnis wird das Längenverhältnis der Folie nach zu vor der Reckung verstanden.

Das Extrudat der Folie kann mit an sich bekannten Füllstoffen oder Strukturbildnern versehen sein, beispielsweise mit anorganischen Partikeln, wie z.B. Kreide, Talk oder Kaolin. Dadurch kann durch die Reckung in an sich bekannter Weise eine mikroporöse Struktur erzeugt werden mit dem Vorteil einer verbesserten Atmungsaktivität.

Die Folie kann aber auch vor der Reckung mit an sich bekannten Methoden perforiert worden sein, so dass sich die Perforationen nach dem Recken zu größeren Perforationen ausweiten.

Die Folie kann aber auch vor dem Recken geschlitzt worden sein, so dass insbesondere durch Reckung im 90° Winkel zu der Längenausdehnung der Schlitze dieselbe zu Perforationen ausgeweitet werden.

Die Folie kann vor der Reckung musterartig geschwächt worden sein, so dass die geschwächten Stellen beim Recken zu Perforationen aufgeweitet werden. Die musterartige Schwächung der Folie kann durch eine Kalanderwalzenpassage, d.h. durch Hitze und Druck bzw. durch Ultraschall-Behandlung erfolgen.

Die Folie kann unabhängig, ob perforiert, musterartig geschwächt oder geschlitzt, aus einer einzigen Schicht bestehen oder durch Coextrusion aus mehreren Schichten, d.h. mindestens zwei Schichten aufgebaut sein. Einer der beiden oder die beiden äußeren Schichten der coextrudierten Folie können aus niedriger schmelzenden Thermoplasten als die andere bzw. die Mittelschicht bestehen. Die Fasern der die Schrumpffolie umgebenden Vliessstofflagen können ausschließlich an der bzw. den niedriger schmelzenden Schicht(en) der coextrudierten Folie und nicht an der Mittelschicht angebunden sein.

Vorzugsweise besteht die Folie aus einer einzigen Polymerkomponente oder aus mindestens zwei Schichten mit einem höher und einem tieferschmelzenden Polymer, und ist durch Coextrusion erzeugt worden. Vorzugsweise ist der Schmelz- oder Erweichungsbereich der Folie bzw. der niedriger schmelzenden Schicht der coextrudierten Folie sehr ähnlich dem Schmelz- oder Erweichungsbereich der Fasern des Vliesstoffes.

Vorzugsweise bestehen Material der Folie und der Fasern des Vliesstoffes aus der gleichen Polymerklasse.

Bevorzugte Materialkombinationen sind Vliesstoff aus Polypropylen oder Copolyproyplen und Folie aus Polypropylen oder einem Copolymer aus Propylen mit einem anderen Olefin oder einem Verschnitt aus Polypropylen und Polyethylen.

Durch den Verstreckungsgrad der Folie kann der Schmelz- oder Erweichungsbereich derselben an die der Endlosfasern im Vliesstoff angeglichen werden. Es könnte beispielsweise auch ein Polyethylen-Spinnvliesstoff aus Polyethylen hoher Dichte (HDPE) oder Polyethylen niedriger Dichte (LLDPE) als eine Schicht und eine blasgeformte und damit nur sehr wenig verstreckte PP-Folie oder eine gegossene, unverstreckte PP-Folie miteinander verschweißt werden, weil sich die Verschweißtemperaturen durch die stark unterschiedlichen Verstreckungsgrade des Spinnvliesstoffes und der Folie weitestgehend angleichen.

Die Erfindung schließt auch die Verwendung einer hydrophoben monolithischen, d.h. nicht wasserdampfdurchlässigen Folie ein.

Für die meisten Anwendungen des elastischen Verbundstoffes wird jedoch aus Gründen des besseren Tragekomforts und zur Verhinderung von Mazeration der Haut eine wasserdampfdurchlässige Materialkombination bevorzugt.

Bekanntermaßen sind mikroporöse Folien aus hydrophobem Polymermaterial bzw. hydrophober Nachausrüstung wasserdampfdurchlässig und für spezielle Ausführungsformen dieser Erfindung besonders begünstigt.

Auch die höhere Weichheit und hohe Opazität der mikroporösen Folien sprechen für den bevorzugten Einsatz als eine Schicht des erfindungsgemäßen Verbundstoffes.

Für Anwendungen in einer Einweg-Windel (Disposable) sind Mikroporösfolien aus hydrophoben Polyolefinen und Copolymeren derselben miteinander vorteilhaft.

Polyolefin-Folien, deren Mikroporosität durch Einlagerung von mit Stearinsäure überzogenen mineralischen Füllstoffen, wie Kreide, in das Polymer und Reckung erzeugt worden sind, eignen sich besonders für den Einsatz in Einmalwindeln.

Auch hier können die Methoden der Coextrusion zur Folienherstellung zur Anwendung kommen.

Die mikroporösen Polyolefin-Folien werden vorzugsweise durch Variation im Reckungsverhältnis (je höher gereckt, desto höher der Schmelz- oder Erweichungsbereich) den Schmelz- oder Erweichungsbereichen der Vliesstoff-Schicht angepasst. Die Reckung kann in Maschinenlaufrichtung, quer zur Maschinenlaufrichtung oder in beiden Richtungen erfolgt sein. Im Sinne einer maximalen Mikroporosität kann es aber auch umgekehrt von Vorteil sein, die Folie stark zu verstrecken und das Faserpolymer der Vliesstoffschicht durch Copolymerisation an die Schmelz- bzw. Erweichungsbedingungen der mikroporösen Folie anzugleichen.

Die Folie kann genauso wie der Vliesstoff weiß- oder farbig pigmentiert sein zum Zwecke der Opazitätserhöhung und/oder der Farbgebung .

Eine besonders bevorzugte Variante der Erfindung ist die Kombination segmentierter Polyester- oder -Polyetherurethanharnstoffe für die Herstellung der elastischen Fäden und Polyolefinfasern für die Vliesbildung der beiden Lagen.

Als Schicht einer Verbundstruktur können auch uni- oder biaxial gereckte, extrudierte Plastiknetze verwendet werden. Der Verstreckungsgrad in beiden Richtungen kann gleich oder unterschiedlich sein.

Vorzugsweise ist jedoch zumindest eine Vorzugsrichtung stark verstreckt. Unter starkem Verstreckungs- oder Reckgrad wird ein Verstreckungsverhältnis von mindestens 3 : 1 verstanden.

Die Stärke der Fäden liegt üblicherweise bei 150 bis 2000 μm. Unter extrudierten Plastik-Netzen werden Flächengebilde mit Gitterstruktur verstanden, die dadurch gebildet wird, dass sich erste parallel angeordnete Monofilamentscharen mit zweiten ebenfalls parallel angeordneten Monofilamentscharen in einem bestimmten gleichbleibenden Winkel kreuzen und an den Kreuzungspunkten miteinander eigenverschweißt sind. Bei Plastiknetzen bestehen die beiden Monofilamentscharen im Normalfall aus demselben Polymer. Die Dicke und der Verstreckungsgrad der zwei Filamentscharen kann aber unterschiedlich sein.

Als weitere Flächengebilde können auch Gelege eingesetzt werden, die sich von Plastiknetzen oder- Gittern dadurch unterscheiden, dass die sich kreuzenden Filamentscharen an ihren Kreuzungspunkten nicht durch Eigenbindung miteinander verbunden sind sondern durch eine Bindemittelapplikation, wie beispielsweise wässrige Polymerdispersionen. In diesem Fall können die beiden parallel orientierten Monofilamentscharen aus unterschiedlichem Polymer bestehen. Bei Gelegen können sowohl gereckte Monofilamentfäden als auch Homofilamente zum Einsatz kommen. Der Winkel der sich kreuzenden Filamentscharen kann prinzipiell beliebig sein. Der Winkel von 90° wird aus praktischen Gründen jedoch bevorzugt. Die Filamentscharen des Geleges oder Plastiknetzes sind vorzugsweise parallel, in Maschinenlaufrichtung und die zweiten Filamentscharen quer d.h. im 90° Winkel zu Maschinenlaufrichtung ausgerichtet. Der Abstand zwischen den ersten parallel in Maschinenlaufrichtung ausgerichteten Filamenten liegt üblicherweise im Bereich zwischen ca. 0,5 und ca. 20 mm, vorzugsweise zwischen 2 und 10 mm und die der zweiten parallel ausgerichteten Filamentscharen zwischen 3 und 200 mm.

Neben den bereits beschriebenen weiteren Flächengebilden können auch Gewebe und Gewirke eingesetzt werden.

Die erfindungsgemäß eingesetzten elastischen Fäden können beliebiger Natur sein, solange diese elastischer Natur sind und mit dem Material der sie umgebenden Vliesstoffschichten bzw. Schichten aus weiteren Flächengebilden in gespanntem Zustand eingebettet werden können. Typischerweise werden die elastischen Fäden mit den sie umgebenden Schichten an den Verschweißungsstellen nicht thermisch verbunden, sondern werden durch die Verschweißung der beiden Schichten in gespanntem Zustand mechanisch fixiert. Es kann jedoch auch Materialkombinationen geben, in denen die elastischen Fäden mit dem Material der sie umgebenden Schichten an den Verschweißungsstellen Verbindungen eingehen. Bevorzugt werden die elastischen Fäden an diesen Stellen jedoch lediglich mechanisch fixiert.

Als elastische Fäden können Monofilamente, Stapelfasergarne oder Multifilamentgarne aus Endlosfilamenten zum Einsatz kommen. Die Garne können als Glattgarne eingesetzt werden oder in verzwirnter Form.

Die erfindungsgemäß eingesetzten elastischen Fäden können aus unterschiedlichen elastomeren Materialien bestehen.

In der Regel handelt es sich dabei um elastomere Kunststoffe. Beispiele dafür sind Elastomere auf Basis von Blockpolyetheramiden, Blockpolyetherestern, Polyurethanen, Polyurethanharnstoffen, elastischen Polyolefinen, thermoplastischem Styrol-Butadien-Styrol, Styrol-Isopren-Styrol, Styrol-Ethylen/Propylen-Styrol, Styrol-Ethylen-Butadien-Styrol, hydriertem Styrol-Butadien-Gummi und deren Verschnitten mit andern Polymeren, wie z.B. mit Polystyrol oder mit Polyolefinen.

Vorzugsweise werden elastische Fäden aus segmentierten Polyester- oder Polyetherurethan-Harnstoffen eingesetzt. Diese sind vorzugsweise aus Dimethylacetamid oder Dimethylformamid-Lösung ersponnen.

Je nach Anforderung an die Retraktionskraft können auch aus der Schmelze gesponnenen elastomere Thermoplastfäden eingesetzt werden, beispielsweise elastomere Polyurethane, elastomere Polyester oder elastomere Polyamide.

Die elastischen Fäden können vor oder nach der Laminierung zu einem Verbundstoff nachvernetzt worden sein, wodurch das Elastomer bis zu einem gewissen Umfang in einen Duroplast umgewandelt worden ist.

Die Scharen von parallel verlaufenden elastischen Fäden weisen einen Abstand der einzelnen Fäden von typischerweise jeweils 0,5 bis 15,0 mm, vorzugsweise 1,0 bis 10,0 mm auf.

Der Titer der elastischen Fäden liegt typischerweise im Bereich von 22 bis 500 dtex, vorzugsweise im Bereich von 44 bis 300 dtex.

Die elastischen Fäden können durch elastische Folienbändchen ersetzt werden.

Als Ausgangsmaterial hierzu dient eine Elastomerfolie, die entweder aus einer Polymerlösung gegossen oder durch CastExtrusion aus einer Elastomerschmelze hergestellt worden ist. Die Folie wird in Bändchen geschnitten, die vorzugsweise in Maschinenlaufrichtung zwischen die beiden Lagen, vorzugsweise zwei Vliesstoffe, in gedehnter Form und parallel zueinander ausgerichtet eingebunden wird. Die Folienbändchen können dabei plan (parallel) oder in einem beliebigen Winkel von 0 bis 180° zwischen die beiden Lagen einlaufen.

In den Fällen, wo die Bändchenbreite gleich oder größer als der Abstand der Folienmitten im Verbundstoff ist, wird ein Ausrichtung des Bändchens in die Lotrechte bevorzugt.

Nach der Kalander-Verschweißung der beiden Lagen, vorzugsweise von PP-Spinnvliesstofflagen, können die Folienbändchen planar dazwischen eingelagert sein oder in einer mehr oder weniger stark gefältelten Form vorliegen.

Die Zuführung der parallel zueinander ausgerichteten Folienbändchen erfolgt üblicherweise über einen Kamm. Ist der Abstand der Kammzähne schmaler als die Bändchenbreite, so fältelt sich das Folienbändchen oder stellt sich in die Lotrechte auf.

Die Folien für die Folienbändchen-Fertigung weisen typischerweise Flächengewichte von 10 - 400 g/m$^2$, vorzugsweise 20 - 200 g/m$^2$ auf.

Die Folienbreite beträgt typischerweise 4 - 20 mm, vorzugsweise 4 - 10 mm.

Daraus errechnen sich Folienbändchentiter von 40 bis 8.000 dtex, vorzugsweise 80 bis 4.000 dtex.

Der Abstand der gefälteten oder ungefältelten Folienbändchenmitte zur Mitte des benachbarten (nächstfolgenden) Folienbändchens quer zur Maschinenlaufrichtung beträgt typischerweise 2 bis 30 mm, vorzugsweise 5 bis 15 mm. Dieser Abstand macht üblicherweise höchstens die Hälfte der Folienbreite aus. Dies entspricht einem Flächengewicht des Folienbändchenanteiles von wenigstens 20 g/m$^2$ und höchstens 800 g/m$^2$, vorzugsweise jedoch 40

g/m$^2$ bis 400 g/m$^2$.

Die Laufrichtung der Fadenscharen stimmt vorzugsweise mit der Maschinenlaufrichtung überein.

Der erfindungsgemäße elastische Verbundstoff besteht aus zumindest zwei Lagen, insbesondere zwei Vliesstoff- oder Faserflorlagen und, vorzugsweise in Maschinenlaufrichtung ausgerichteten, parallel verlaufenden elastomeren Fäden oder Garnen zwischen den beiden Lagen, wobei der Verbund aus dem starren, unelastischen Vliesstoff und den gedehnten elastischen Fäden ohne Verwendung eines zusätzlichen Klebstoffes (Schmelzkleber oder Haftkleber) erfolgt.

Typischerweise weist der erfindungsgemäße Verbundstoff im vollgedehnten, d.h. plissierungsfreien Zustand, Flächengewichte von 15 bis 150 g/m$^2$, vorzugsweise 15 bis 70 g/m$^2$, auf.

Die maximale Dehnung m des elastischen Verbundstoffes bis zum plissierungsfreien Zustand liegt typischerweise im Bereich von 10 bis 350%, vorzugsweise 20 bis 250%.

Die Flächengewichte des voll entspannten (relaxierten) Verbundstoffes erstrecken sich typischerweise von 16,5 bis 680 g/m$^2$, vorzugsweise jedoch von 22 bis 350 g/m$^2$.

In einer bevorzugten Ausführungsform besteht der erfindungsgemäße Verbundstoff aus zwei Schichten, wobei beide Schichten aus Vliesstoffen bestehen, zwischen denen die parallel verlaufenden elastischen Fäden eingelagert sind.

In einer weiteren bevorzugten Ausführungsform besteht der erfindungsgemäße Verbundstoff aus mindestens drei Schichten, wobei zwei dieser Schichten aus Vliesstoffen bestehen, zwischen denen die parallel laufenden elastischen Fäden eingelagert sind und mindestens einer einen der Vliesstoffe abdeckenden Schicht, die vorzugsweise ein Stapelfaser-Vliesstoff ist.

Die beiden Lagen des erfindungsgemäßen Verbundstoffes, zwischen denen die elastischen Fäden eingelagert sind, bestehen zumindest anteilig aus Fasern desselben Faserpolymers, wobei der Fasertiter unterschiedlich sein kann.

Vorzugsweise bestehen die beiden Lagen jeweils vollständig aus den gleichen schmelzgesponnen Fasern, die entweder als Kurzschnitt-, als Stapel- oder als Endlosfasern abgelegt worden sind.

Bei der Auswahl der Polymerkombinationen für den erfindungsgemäßen Verbundstoff ist darauf zu achten, dass das Elastomer der Fäden mit dem Polymer der Vliesstoffschicht bzw. der Schicht des weiteren Flächengebildes im wesentlichen keine thermische Bindung eingeht und dass die thermische Verschweißung bevorzugt lediglich zwischen den beiden Flächengebilden erfolgt.

Bevorzugte Kombinationen von Fadenelastomer und Faser in Vliesstoff bzw. weiterem Flächengebilde sind in der nachstehenden Tabelle aufgelistet.

| Fadenelastomer | Polymer in Vliesstoff/Flächengebilde |
|---|---|
| Polyurethane | Polyolefine |
| Polyesterelastomere | Polyolefine |
| SBS[1] und/oder SEBS[2] | Copolyester |
| SBS und/oder SEBS (Kraton) | CoPolyamid |

[1] Styrol-Butadien-Styrol

[2] Styrol-Ethylen-Butadien-Styrol

Polyolefine, Copolyester und Copolyamid können auch den niedriger schmelzenden Anteil einer Bikomponentenfaser bilden.

Es war völlig überraschend, dass die gedehnten elastischen Fäden trotz deren Haftungsfeindlichkeit mit dem Polymer der sie umgebenden Schichten der beiden Flächengebilde an deren Verschweißzonen so stark eingequetscht wurden, dass selbst nach zahlreichen wiederholten Dehnungs-/Entlastungszyklen keinerlei Loslösung der elastischen Fäden erfolgte.

Die Fasern der Vliesstoffschicht(en) bzw. das Material des weiteren Flächengebildes müssen thermisch verschweißbar sein. Darunter sind solche Fasern bzw. Materialien zu verstehen, die durch unter Hitze und Druck, Ultraschall sowie Infrarot Energie miteinander verschmelzen oder eine Bindung eingehen.

Die Schmelz- oder Erweichungstemperaturen der Materialien der beiden Lagen, beispielsweise der Faserschichten, müssen tiefer liegen als die der elastischen Fäden, typischerweise um mindestens 25°C tiefer.

Es ist auch denkbar, den elastischen Faden vor der Verbundstoffherstellung einer Vernetzung zu unterwerfen, um entweder dessen Schmelzpunkt zu erhöhen oder das Elastomer gar in einen unschmelzbaren Zustand überzuführen.

Das Verschweißen zwischen Vliesstoff und Flächengebilde und das dadurch hervorgerufene Einklemmen der parallel verlaufenden elastischen Fäden des erfindungsgemäßen Verbundstoffes erfolgt vorzugsweise durch Hitze und Druck im Kalanderspalt und/oder durch Ultraschall.

Werden schrumpffähige Vliesstoffe und/oder Flächengebilde eingesetzt, so kann die Schrumpfung dabei nur in einer Vorzugsrichtung erfolgen, aber auch in beiden oder in mehr als zwei Richtungen. Die Schrumpfbeträge bei mehreren Richtungen, wie in beiden Richtungen, d.h. in Maschinenlaufrichtung und im neunzig Gradwinkel zur Maschinenlaufrichtung, können gleich oder völlig unterschiedlich sein.

Der erfindungsgemäße Verbundstoff kann aus einem Vliesstoff und einem damit in Form eines vorgegebenen Musters thermisch verschweißten weiteren Flächengebilde bestehen, zwischen denen parallel verlaufende elastische Fäden in gespanntem Zustand eingebettet sind.

Das weitere Flächengebilde kann aber auch auf beiden Seiten mit einem Vliesstoff abgedeckt sein, entweder symmetrisch oder asymmetrisch, d.h. die Gewichte beiden Vliesstofflagen können unterschiedlich oder gleich sein. Zumindest zwischen einer Vliesstofflage und dem weiteren Flächengebilde, das ebenfalls ein Vliesstoff sein kann, sind parallel verlaufende elastische Fäden in gespanntem Zustand zwischen den umgebenden Schichten eingebettet. An den Verschweißzonen werden die gedehnten elastischen Fäden zwischen den beiden Flächengebilden zerstörungs- bzw. schädigungsfrei eingequetscht, ohne eine Schmelzhaftung mit ihnen einzugehen. Zwischen der oberen und der unteren Vliesstoffschicht und dem weiteren Flächengebilde können sich auch zwei Scharen von jeweils parallel verlaufenden elastischen Fäden befinden, die in gespanntem Zustand in die umgebenden Schichten eingebettet sind. Dabei können die Richtungen der Fadenscharen gleich sein oder sich voneinander unterscheiden.

Das Muster der thermischen Verschweißung der Vliesstoffe bzw. Flächengebilde zur Fixierung der elastischen Fäden zwischen den darüber und darunter liegenden Flächengebilden des erfindungsgemäßen Verbundstoffes kann beliebig sein, solange es eine vollständige Immobilisierung der elastischen Fäden in gespanntem Zustand in den Verschweißungszonen der diese Fäden umgebenden Flächengebilde gestattet. Gravurgeometrien mit in Reihe ausgerichteten einzelnen Punkten oder anderen beliebigen Formen sind dann nicht geeignet, wenn die Inreihe-Ausrichtung derselben parallel, d.h. im Normalfall in Maschinenlaufrichtung, zur Ausrichtung der elastischen Fäden gewählt wird, da sich mit diesem Verlauf der Verschweißungs-muster keine Fixierung der elastischen Fäden in gespanntem Zustand zwischen den beiden Flächengebilden erreichen lässt.

Bevorzugte Muster für eine thermische Verschweißung sind durchgehende Linien unterschiedlicher Breite in paralleler Anordnung. Es sind aber auch andere Muster denkbar, die zu rauten-, wellen-, zickzack- oder kreisförmigen Verschweißzonen führen.

Die beide Lagen verbindende Verschweißflächen liegen typischerweise im Bereich von 10 - 40 %, vorzugsweise 15 - 30 %, bezogen auf die Gesamtfläche.

Der erfindungsgemäße Verbundstoff weist im voll verdehnten Zustand in der Regel eine zweidimensionale Struktur auf. Bei Entspannung bildet sich eine dreidimensionale Struktur aus. Es entstehen Plissierungen, deren Form, Abstand und Höhe innerhalb weiter Grenzen durch das Gravur-Dessin und den Verdehnungsgrad verändert werden kann.

Der erfindungsgemäße Verbundstoff zeichnet sich gegenüber dem Stand der Technik dadurch aus, dass er in Richtung der Schar der parallel verlaufenden elastischen Fäden, in der Regel also in Maschinenlaufrichtung, unelastische und elastische Bereiche in sich wiederholender Anordnung aufweist. Der elastische Bereich ist der Bereich zwischen jeweils zwei benachbarten Verschweißlinien auf einem elastischen Faden. Die elastischen, gedehnten Fäden sind in den unelastischen Bereichen mechanisch eingequetscht und werden in die sie umgebenden beiden Schichten eingebettet.

Die Retraktionskraft des Verbundstoffes kann durch Veränderung des Titers der Elastomerfäden und deren Abstand zueinander stark variiert werden.

Die Erfindung betrifft auch ein Verfahren zur Herstellung des weiter oben beschriebenen Verbundstoffes umfassend die Maßnahmen:

a) Kombination von mindestens einem Vliesstoff mit einem weiteren Flächengebilde und einer zwischen dem Vliesstoff und dem weiteren Flächengebilde in gespanntem Zustand angeordneten Schar parallel verlaufender elastischer Fäden, und

b) Einschweissen der Schar parallel verlaufender elastischer Fäden zwischen dem Vliesstoff und dem weiteren Flächengebilde in Form eines vorbestimmten Musters, vorzugsweise durch Hitze und Kalanderdruck und/oder durch Ultraschall, so dass ausgewählte Bereiche jedes elastischen Fadens in gespanntem Zustand an den Verschweißungsstellen zwischen den Vliesstoff und das weitere Flächengebilde eingebettet werden.

Das thermische Verschweißen von Vliesstoff und dem weiterem Flächengebilde kann auf beliebige Arten erfolgen, beispielsweise durch Kalandrieren mit einem Prägekalander, dessen eine Walze ein vorbestimmtes Muster, vorzugsweise ein regelmäßiges Linienmuster aufweist, oder durch Verschweißen mit Ultraschall oder mit Infrarotstrahlung, die jeweils in einem vorbestimmten Muster auf den Vliesstoff und das weitere Flächengebilde einwirken.

Die Schar von elastischen Fäden kann in beliebiger Richtung zur Maschinenlaufrichtung verlaufen. Bevorzugt verläuft sie parallel in Maschinenlaufrichtung.

Die parallel zueinander ausgerichteten über die gesamte Warenbreite verteilten elastischen Fäden sind derart zwischen zwei Lagen von Vliesstoff bzw. weiterem Flächengebilde eingelagert, dass die elastischen Fäden entlang vorgegebener Abschnitte selbst keine Haftung zu dem Material der beiden Lagen aufweisen und mit den Lagen nur in vorgegebenen Verschweißstellen verbunden sind, vorzugsweise mit dem Material der beiden Lagen entlang durchgehender, ununterbrochener Verschweißlinien miteinander verbunden sind.

Die Verschweißlinien können im Prinzip jede gewünschte Form aufweisen und bilden typischerweise einen Winkel zwischen 45 und 90° zu den parallel ausgerichteten elastischen Fäden. Der Winkel kann, muss aber nicht an allen Stellen gleich sein.

Beim erfindungsgemäßen Verfahren werden die elastischen Fäden oder die Folienbändchen im gedehnten Zustand zwischen die beiden Lagen, vorzugsweise die beiden Vliesstofflagen, platziert.

Der Betrag der gewünschten Dehnung kann durch Differenzgeschwindigkeit der Zuführ- und Abzugsvorrichtungen der Fertigungsvorrichtung, beispielsweise des Fadenabzuges und der Kalanderwalzen, eingestellt. Die elastischen Fäden können auf Kettbäumen oder Teilkettbäumen aufgewickelt sein. Der Abzug kann aber auch aus Spulen, die auf einem Gatter stecken, abgewickelt und einem Kalanderspalt zugeführt werden.

Oberhalb und unterhalb der gedehnten Fäden oder Folienbändchen werden die beiden Lagen, vorzugsweise aus prägegebundenen Polyolefin-Spinnvliesstoffe, einem Kalander-Pressspalt zugeführt.

In einer bevorzugten Ausführungsform ist eine Walze mit einer glatten Oberfläche und die andere mit einer kontinuierlichen Linienprägung versehen. Im Falle eines ungleichen Gewichtes der beiden Lagen wird diejenige mit dem niedrigeren Flächengewicht in Kontakt zu der Glattwalze gebracht.

Bevorzugt sind die Kanten der linienförmigen Gravur leicht abgerundet. Dadurch wird sichergestellt, dass ein Abschneiden oder Durchtrennen der im Kalanderspalt gepressten elastischen, gedehnten und erwärmten Fäden bzw. Folienbändchen wirksam verhindert wird.

Die Herstellung des elastischen Verbundstoffes kann neben der Kalandrierung in einem Kalander durch Hitze und Druck auch mithilfe der Ultraschalltechnik bewerkstelligt werden.

Die Ware kann nach der Kalandrierung zu einem elastischen Verbundstoff im gedehnten Zustand aufgewickelt werden. Es ist jedoch vorteilhaft, die Ware nach dem Kalanderdurchgang zu relaxieren (z.B. durch eine oder mehrere langsamer als die Kalanderwalzen laufenden Walzen) und in diesem entspannten Zustand den Verbundstoffes einer Wasserdampfbehandlung entsprechend der gängigen Praxis für elastische Textilien zu unterziehen, zum Zwecke eines Nachschrumpfens der elastischen Fäden, einer Vergleichmäßigung der Schrumpfkraft über die gesamte Warenbreite und Warenlänge aber auch zum Zwecke der Entfernung etwaiger in den elastischen Fäden vom Spinnprozess verbliebenen Lösungsmittelanteile und Avivagen.

Nach dieser Nachbehandlung wird die Ware vorzugsweise wieder im gespannten Zustand aufgewickelt.

Der erfindungsgemäße Verbundstoff lässt sich insbesondere zur Fertigung von Hygieneartikeln, insbesondere von Windeln einschließlich Windelhöschen, einsetzen. Diese Verwendung ist ebenfalls Gegenstand der vorliegenden Erfindung.

Kurzbeschreibung der Zeichnung

Die nachfolgenden Abbildungen erläutern die Erfindung näher.

Abbildung 1 beschreibt eine Form des erfindungsgemäßen Verbundstoffes

Abbildung 2 beschreibt den Verbundstoff gemäß Abbildung 1 im Querschnitt entlang der Linie A-A

Abbildung 3 beschreibt den Verbundstoff gemäß Abbildung 1 in entlastetem Zustand im Querschnitt entlang der Linie B-B

Abbildung 4 beschreibt eine Vorrichtung zur Herstellung des erfindungsgemäßen Verbundstoffes.

Ausführung der Erfindung

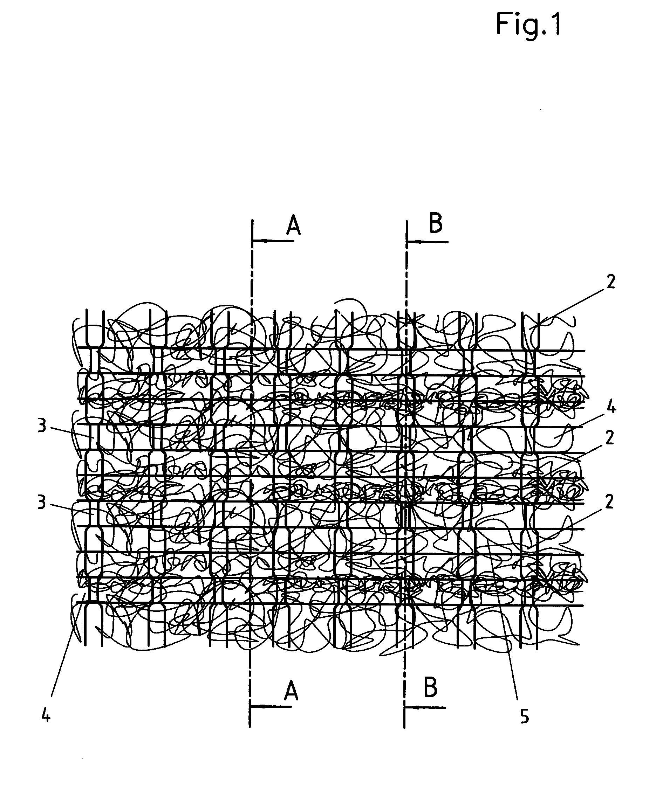

Eine der zahlreichen Varianten des erfindungsgemäßen Faserflächengebildes wird in Abb. 1 schematisch dargestellt. In diesem Fall besteht der Verbund aus insgesamt drei Vliesstofflagen. Der Verbundstoff (1) ist in Aufsicht dargestellt, wobei die zwischen den Vliesstoffschichten eingelagerten elastischen Fäden zum besseren Verständnis eingezeichnet worden sind.

Die Fasern (5) der beiden Vliesstoffschichten oder Faserflore sind entlang der Verschweißlinien (4) intensiv miteinander verschmolzen (autogen verschweißt).

Die in Abbildung 1 dargestellte Version des Verbundstoffes befindet sich im relaxierten (d.h. entlasteten) Zustand der eingelagerten elastischen Fäden. Dadurch, dass die elastischen Fäden im gedehnten Zustand mit den beiden starren, unelastischen Vliesstofflagen zu einem Verbundstoff vereinigt worden sind, entstehen bekanntermaßen nach der Entspannung der Fäden dreidimensionale Strukturen mit einer Plissierung beidseitig der Fadenfläche.

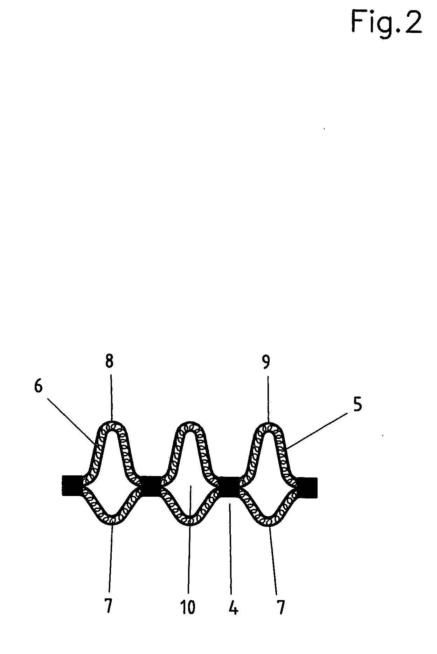

Diese ist in Abbildung 2 durch den Querschnitt entlang der Linie A---A dargestellt.

Die beiden Vliesstofflagen (6) und (7) werden nach der Entspannung gewellt mit den Scheitelpunkten (8) und (9) beidseitig symmetrisch zu den Verschweißstellen (4). Durch diese Wellungen bilden sich Hohlräume (10) aus.

In Abbildung 1 wird deutlich, dass der, vorzugsweise in Maschinenlaufrichtung ausgerichtete, elastische Faden im total oder teilentspannten Verbundstoff abwechselnd Bereiche höherer Dicke (Titer) und geringerer Dicke (Titer) aufweist.

Innerhalb der Verschweißbereiche (4) ist der elastische Faden so stark mechanisch eingequetscht, dass sein gedehnter Zustand, d.h. die Dicke (3) während der Fertigung des Verbundstoffes eingefroren bleibt, völlig im Gegensatz zu den Bereichen des elastischen Fadens zwischen den Verschweißzonen (4), wo der Faden weitgehend ungehindert durch den Vliesstoff eine dem Grad der Entlastung entsprechende höhere Dicke (2) einnehmen kann.

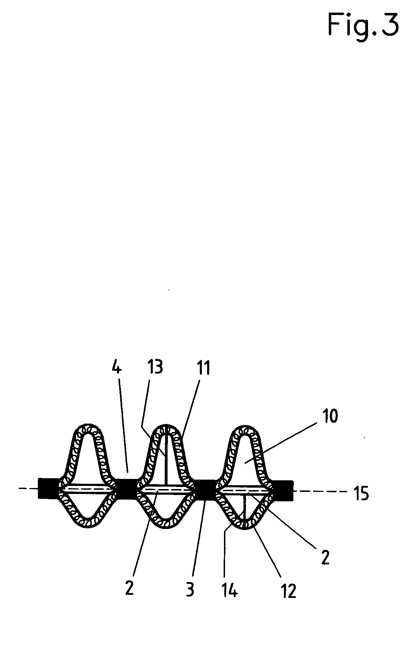

In Abbildung 3 ist der Querschnitt des elastischen Verbundstoffes im entlasteten Zustand entlang der Linie B-B widergegeben. In den verschweißten Stellen (4) ist der elastische Faden mit seinen Dünnstellen (=gedehnter Zustand) (3) und seinen Dickstellen (2) (teil- bis voll entlasteter Zustand) dargestellt. Außerhalb der Verschweißstellen (4) werden die Wandungsdicken (11) des oberen und die Wandungsdicken (12) des unteren Vliesstoffes durch deren Gewicht und Verfestigungsbedingungen bestimmt. Die Schenkelhöhen (12) bzw. (13) der Wellungen beidseitig der Fadenebene können gleich oder ungleich sein und hängen weitgehend von den Herstellungsbedingungen, z.B. von den eingesetzten Walzenkalanderpaarungen, ab. Werden im Falle einer Verfestigung mit Wärme und Druck eine glatte und eine gravierte Kalanderwalze eingesetzt, so sind die Schenkelhöhen des entspannten Verbundstoffes auf der in der die Gravur eingeprägt wird, größer als die Gegenseite. Unter Scheitelhöhen (13) bzw. (14) wird im Rahmen dieser Beschreibung der Abstand zwischen den Scheitelpunkten (8) bzw. (9) und der Fadenebene (15) verstanden.

Die Hohlräume 10 verkleinern sich umso mehr, je höher die prozentuale Kontraktion (Relaxation) ist und je weicher und leichter die undehnbaren Vliesstoffe sind. Insbesondere bei hoher Relaxation, entsprechend einer hoher Vorspannung des elastischen Fadens bei der Verbundstoffherstellung kann der Hohlraum vollständig verschwinden und insbesondere bei leichten und weichen Vliesstoffschichten die Wellungen umklappen. Dieses Umklappen kann durch Pressung gezielt erreicht werden.

Die Verschweißbereiche (4) erscheinen bei Einsatz nicht mit Weißpigment gefüllter Fasern weitgehend transparent. Bei Anwendungen, die eine hohe Opazität erlauben ist es vorteilhaft sowohl für den Vliesstoff als auch die elastischen Fäden eine möglichst hohe Weißpigmentierung, wie beispielsweise aus Titandioxid, zu verwenden. Als weitere günstige Zusatzmaßnahme zur Erhöhung der Opazität bietet sich an, die Wellungen durch Kalandrierung flachzudrücken und damit die transparenteren Schweißstellen abzudecken.

Dem Fachmann ist bekannt, dass diese Abflachung des Verbundstoffes ohne Hitzeeinwirkung bzw. bei solch niedrigen Temperaturen erfolgen sollte, dass ein Verkleben der abgeklappten Vliesstofffältelungen nicht erfolgen kann, denn diese würden die Elastizität des Verbundstoffes vermindern.

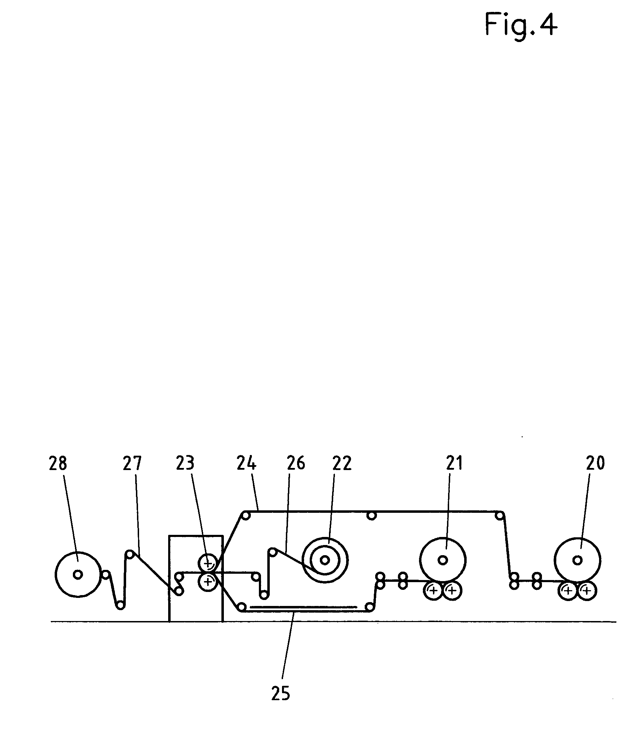

Die in den Abbildungen 1 bis 3 dargestellten Verbundstoffe können in einer Vorrichtung nach Abbildung 4 hergestellt werden.

Die Abrollungen (20) und (21) betreffen in diesem Fall zwei Spinnvliesstofflagen und die Abrollung (22) stellt eine Kettbaumabrollung mit Fadenwächter dar (beispiels-weise mit ca. 50.000 m). Dargestellt ist ferner ein Kalander (23), dem die Vliesstoffe (24) und (25) sowie die Schar parallel verlaufender elastischer Fäden (26) zugeführt und durch Einwirkung von Hitze und Druck an vorbestimmten Stellen miteinander verbunden werden. Der hergestellte Verbundstoff (27) wird auf die Rolle (28) aufgewickelt.

Die nachstehenden Beispiele erläutern die Erfindung ohne diese zu begrenzen.

Beispiel 1:

Von einem Teilkettkaum, der für eine Kette für 18 Fäden/inch (entsprechend 18 Fäden / 2,54 cm) vorbereitet worden war, wurden Elastanfäden auf Basis von in Dimethylacetamid lösungsmittelgesponnenem segmentiertem Polyetherurethan mit einem Titer von 78 dtex, die mit einer Vordehnung von 40% aufgewickelt worden waren, mit einer Geschwindigkeit von 2,5 m/min abgewickelt.

Die parallele Fadenführung erfolgte mittels zweier Rechen für 18 Fäden/inch (entsprechend 18 Fäden / 2,54 cm), wobei die beiden Rechen im 90° Winkel zur Maschinenlaufrichtung ausgerichtet waren. Einer der beiden 50 cm breiten Rechen wurde unmittelbar hinter dem Kettbaum und der zweite vor den beiden Kalanderwalzen installiert. Die Rechen wurden einer Raschelmaschine entnommen. Die parallel in Maschinenlaufrichtung ausgerichteten Elastanfäden wurden dem Pressspalt (Nip) einer glatten Kalanderwalze und einer gravierten Kalanderwalze - jeweils aus Edelstahl - zugeführt bzw. eingeklemmt. Die Geschwindigkeit der Kalanderwalzen betrug 5 m/min. Als Gravurwalze wurde eine solche mit linienförmigen Erhebungen beinahe quer zur Maschinenlaufrichtung eingesetzt, die im Folgenden mit Line Seal Gravurwalze bezeichnet wird.

Daten der Line Seal Gravurwalze:

Breite der Linien 1,00 mm
Abstand der zweier Linienmitten zueinander: 4,00 mm
Verschweißfläche: 25 %
Gravurtiefe: 0,90 mm
Winkel der Linien gemessen quer zur Maschinenlaufrichtung: 0,8°

Die Line Seal Gravurwalze wurde vor dem Einsatz an deren Kanten abgerundet mit dem Hintergrund, ein Abzwicken bzw. Durchtrennen der gedehnten Elastanfäden während der Kalandrierung zu verhindern.

Der Winkel von 0,8° wurde gewählt, um eine Laufruhe der Kalanderwalzen (ohne Stützränder) zu gewährleisten (d.h. ein Rattern der Walzen zu verhindern).

Unmittelbar vor dem Walzenspalt wurden je ein weiß mattierter Polyproyplen-Spinnvliesstoff mit einem Flächengewicht von jeweils 17 g/m$^2$ oberhalb und unterhalb der gespannten Elastanfäden zugeführt und im Kalanderpressspalt zu einem dreilagigen Verbundstoff mit Line Seal Verschweißungen verbunden. Die Polyproyplen-Spinnvliesstoffe wiesen ein ausgeglichenes Verhältnis zwischen Höchstzugkraft in Längs- und Querrichtung auf und waren mit 0,9% Titandioxid zum Zwecke einer Opazitätserhöhung in der Spinnmasse pigmentiert worden.

Die Temperatur beider Walzen betrug 145°C und der Liniendruck 35 N/mm.

Der Verbundstoff wurde mit einer Geschwindigkeit von 5 m/min., d.h. im gespannten Zustand aufgerollt. Nach dem Abrollen und völligen Entspannen (Relaxation) des Verbundstoffes entstand eine Ware mit symmetrischen Plissierungen beidseitig der Elastanfadenfläche und zwischen den Line Seal Verschweißungen.

Es überraschte, dass die Elastanfäden zwischen den Schmelz- bzw. Schweißstellen der beiden Polypropylenspinnvlieslagen selbst bei vielfach wiederholten Dehnungs- und Relaxationszyklen bis zur völligen Entfernung der Plissierung keine Lockerung bzw. kein Ausschlupfen aus den Verbund erlitten.

Dies ist insofern überraschend, als eine hohe Differenz der thermischen Verschweißtemperatur der Polypropylenspinnvlieslagen (hier 145°C) und dem segmentierten Polyurethanharnstoff der Elastanfäden bestehen (Erweichungstemperatur ca. 190-200 °C).

Im gedehnten Zustand (d.h. im plissierungsfreien Zustand der Spinnvliesstofflagen) des in Maschinenlaufrichtung elastischen Verbundstoffes ergaben sich folgende Flächengewichte für die drei Schichten:

| Komponenten des Verbundstoffes | Flächengewicht in g/m$^2$ |
|---|---|
| Polypropylenspinnvlieslage 1 | 17,00 |
| 78 dtex Elastan Garn mit 18 Garnen/inch | 1,974 |
| Polypropylenspinnvlieslage 2: | 17,00 |
| Summe | 35,974 |

Das Flächengewicht F für die Elastangarne errechnete sich nach folgender Beziehung:

$$F = \frac{T*g*100}{10000 * 2,54 * (1 + 0,01 * v) * k/a}$$

Dabei bedeuten

T = Titer des Elastangarnes in dtex
g = Garnteilung in Zahl/inch
v = Vordehnung des Elastangarnes auf dem Kettbaum in %
a = Abrollgeschwindigkeit vom Kettbaum in m/min
k = Kalandergeschwindigkeit in m/min.

Berechnung für Beispiel 1:

t = 78 dtex
g = 18/inch (entsprechend 18 / 2,54 cm)
v =40%
a = 2,5 m/min
k = 5,0 m/min.

Dieses ergibt nach obiger Formel einen Wert für F von 1,974 g/m$^2$.

Der Verbundstoff aus Beispiel 1 wurde im relaxierten Zustand an zwei in Maschinenlaufrichtung beabstandeten Stellen markiert, dann gedehnt bis die Plissierung völlig verschwunden war, und erneut der Abstand der beiden Markierungen gemessen.

Aus dem Verhältnis des Abstandes im gedehnten und ungedehnten Zustandes wurde die Dehnung gemessen, die hier als maximale Dehnung bezeichnet wird.

Im Beispiel 1 lag eine elastische Dehnbarkeit bis max. 95 % vor.

In dem erfindungsgemäßen gemäß Beispiel 1 ist in Maschinenlaufrichtung zwischen zwei sich abwechselnden Bereichen zu unterscheiden. Die Line Seal verschweißten Bereiche, die von der Gesamtfläche einen Anteil von 25 % ausmachten und die Bereiche zwischen den Verschweißlinien, die im Falle einer Dehnung bis zum völligen Verschwinden der Plissierung eine Fläche von 75 % ausmachten.

Die verschweißten Bereiche (25%) sind völlig unelastisch und undehnbar. Der Elastanfaden lag hier in einem Zustand vor, der einer Dehnung auf das 2,8-fache und damit einem Titer von 27,86 dtex entsprach und zwar völlig unabhängig davon, in welchen Dehnungszustand sich der Verbundstoff befand.

Die maximale elastische Dehnbarkeit des Verbundstoffes von nur 95 % (d.h. einer Dehnung auf das 1,95-fache) zeigt deutlich, dass die beiden Polypropylenspinnvlieslagen eine totale Relaxation des Elastanfadens auf seinen Ursprungstiter von 78 dtex verhindern.

Der Flächengewichtsanteil f des relaxierten neuen Verbundstoffes lässt sich aus folgender Beziehung errechnen:

$$f = 0{,}01*w*F + (1+0{,}01*m-0{,}01*w)*F.$$

Dabei bedeuten:

w = Flächenanteil der Verschweißzonen in %
p = Flächenanteil der Bereiche zwischen den Verschweißzonen im maximal (d.h. plissierungsfrei) gedehnten Zustand in % (entsprechend p = 100 - w)
m = maximale Dehnung des Verbundstoffes (bis zum plissierungsfreien Zustand) in %
F = Flächengewicht für die Elastangarne

Die Berechnung für Beispiel 1 ergab:

w =25%
p =75%
m=95%
F = 1,974.

Daraus errechnet sich nach obiger Formel ein Wert für f von

$$f = 1{,}95 - 1{,}974 = 3{,}8493 \text{ g/m}^2 \text{ Elastan-Fäden,}$$

wobei sich f auf die verschweißen und unverschweißten Bereiche $f_v$ und $f_u$ wie folgt verteilt:

$$f_v = 0{,}01*w*F \qquad f_u = f - f_v$$

Dieses ergibt Werte für $f_v$ und $f_u$ von

$f_v = 0{,}25 * 1{,}974 = 0{,}4935$ g/m$^2$
$f_u = 3{,}3558$ g/m$^2$

Um festzustellen in welchen Titer und damit auch in welchen Dehnungszustand der Elastanfaden nach Relaxation im Verbundstoff des Beispieles 1 vorlag, muss folgende Formel angewendet werden:

$$M_p = \left( \frac{1 + 0,01 * m - 0,01 * w}{0,01 * p} - 1 \right) * 100 \ (\%)$$

Dabei bedeuten:

$M_p$ = maximale Dehnung innerhalb der Plissierungsbereiche in % und die übrigen Variablen haben die weiter oben angegebene Bedeutung.

Für Beispiel 1 errechnet sich daraus:

$M_p$ = 126,66 %.

Aufgrund der Tatsache, dass 25 % der Fläche, bezogen auf plissierungsfrei gedehnten Zustand, völlig unelastisch bleiben, reduziert sich der maximale mögliche Dehnungsbetrag von 126,66 % auf 95 % bezogen auf die Gesamtfläche.

Im relaxierten als auch gedehnten Zustand des neuen Verbundstoffes aus Beispiel 1 liegt das Elastangarn in einem Titer $T_d$ von

$$T_d = \frac{T}{(1 + 0,01 * v) * k/a} \, dtex \ vor.$$

Dabei haben die Variablen die weiter oben angegebene Bedeutung.

Für Beispiel 1 errechnet sich somit ein Wert von $T_d$ = 78 / 2,8 = 27,857 dtex.

Für die relaxierten Elastangarnbereiche zwischen den Verschweißzonen des Verbundstoffes errechnet sich der Titer $T_e$ nach folgender Beziehung:

$$T_e = T_d * (1 + 0,01 * M_p).$$

Dabei haben die Variablen die weiter oben angegebene Bedeutung.

Für Beispiel 1 errechnet sich somit ein Wert von $T_e$ = 27,857 * 2,2666 = 63,14 dtex

Die beiden 17 g/m² schweren Lagen aus Polypropylenspinnvliesstoff verhindern also eine totale Relaxation der Elastangarne von 27,857 dtex auf ihren ursprünglichen Zustand mit 78 dtex, sondern bleiben bei einem Titer von 63,14 dtex blockiert, was einem Rücksprung um nur 126,66 % anstelle 180 % entspricht.

Die Flächengewichte des bis zur völligen Verschwinden der Plissierung gedehnten Verbundstoffes betrugen 38 g/m² und im relaxierten Zustand 74 g/m².

Prüfung auf elastisches Verhalten:

Die Prüfung auf zugelastisches Verhalten wurde in Anlehnung an DIN 53 835 Teil 1 und Teil 14 ermittelt.

Hierzu wurden 25 mm breite Streifen aus Beispiel 1 eines Kraft/Dehnungsrelaxationsversuches über drei Zyklen jeweils bis zu einer maximalen Dehnung mit einer Abzugsgeschwindigkeit von 500 mm/min. unterzogen. Die Messung wurde mit einer Kraft vor Weg von 0,05 N/25 mm begonnen. Der 1. Zyklus lief über 20 Sekunden (10 Sekunden für Belastungskurve bis zu 120 % max. Dehnung und weitere 10 Sekunden für die Entlastung). Unmittelbar danach wurde der 2. Hysterese-Zyklus begonnen mit derselben Dauer wie beim 1. Zyklus. Nach einem Verweilen über 60 Sekunden im relaxierten Zustand wurde der 3. Zyklus durchfahren.

In der folgenden Tabelle 1 sind die Zugkräfte Z bei unterschiedlichen Dehnungen von 40, 60 und 100 und 120% wiedergegeben sowie die Dehnung $\varepsilon$ bei 0,05 N/25 mm und 0,1 N/25 mm sowohl in der Belastungs- als auch Entlastungskurve der 3 Zyklen.

Tabelle 1

| Zugkraft Z in N/25 mm bei unterschiedl. % Dehnung | | | | | Dehnung ε in % bei | |
|---|---|---|---|---|---|---|
| Hysterese | 40% | 60 % | 100 % | 120 % | 0,05 N / 25 mm | 0,1 N / 25 mm |
| 1. Zyklus Belastung | 0,36 | 0,51 | 1,11 | 15,81 | 0,1 | 3,1 |
| 1. Zyklus Entlastung | 0,19 | 0,29 | 0,57 | 15,51 | 16,1 | 22,8 |
| 2. Zyklus Belastung | 0,26 | 0,38 | 0,75 | 14,83 | 8,9 | 13,9 |
| 2. Zyklus Entlastung | 0,19 | 0,29 | 0,56 | 14,76 | 16,7 | 24,2 |
| 3. Zyklus Belastung | 0,29 | 0,41 | 0,79 | 14,68 | 0,7 | 10,6 |
| 3. Zyklus Entlastung | 0,19 | 0,29 | 0,56 | 14,59 | 14,59 | 23,4 |

Die Zugkraft Z nach den Entlastungszyklen wird auch als sogenannte Retraktionskraft bezeichnet. Von besonderer Bedeutung für Windelhöschen ist beispielsweise die Retraktionskraft bei 40% Dehnung nach dem 3. Zyklus Entlastung.

Beispiel 2:

Die beiden 17 g/m$^2$ schweren Polypropylen-Spinnvliesstoffe des Beispieles 1 wurden durch zwei leichtere mit einem Gewicht von nur 8 g/m$^2$ ersetzt. Die in Beispiel 1 beschriebenen Bedingungen blieben unverändert.

Im gedehnten Zustand (d.h. im plissierungsfreien Zustand der Spinnvliesstofflagen) der in Maschinenlaufrichtung elastischen Verbundstoffes ergaben sich folgende Flächengewichte für die drei Schichten:

| Komponenten des Verbundstoffes | Flächengewicht in g/m$^2$ |
|---|---|
| Polypropylenspinnvlieslage 1: | 8,000 |
| 78 dtex Elastan Garn mit 18 Garnen/inch | 1,974 |
| Polypropylenspinnvlieslage 2: | 8,000 |
| Summe | 17,974 |

Es wurde eine maximale elastische Dehnung m von 120 % ermittelt, woraus sich für den Verbundstoff im relaxierten Zustand folgende Gewichte ergaben:

| Komponenten des Verbundstoffes | Flächengewicht in g/m$^2$ |
|---|---|
| Polypropylenspinnvlieslage 1: | 17,600 |
| 78 dtex Elastan Garn mit 18 Garnen/inch | 4,343 |
| Polypropylenspinnvlieslage 2: | 17,600 |
| Summe | 39,543 |

Die im Vergleich zu Beispiel 1 eingestellten höhere Werte für m von 120% (Beispiel 1: 95 %) zeigen deutlich, dass bei eine, geringeren Flächengewicht der beiden Polypropylenspinnvlieslagen die gedehnten Elastanfäden nach der Relaxation weiter als bei Beispiel 1 in ihren Ursprungszustand (78 dtex) zurückspringen konnten.

Die Berechnungen für m = 120% ergeben folgende Daten für Beispiel 2:

| | Einheit | Wert |
|---|---|---|
| F | m$^2$ | 4,3428 |
| $f_v$ | /m$^2$ | 0,4935 |
| $f_u$ | G/m$^2$ | 3,8493 |
| $M_p$ | % | 160% |

(fortgesetzt)

|  | Einheit | Wert |
|---|---|---|
| $T_d$ | dtex | 27,857 |
| $T_e$ | dtex | 72,43 |

Die Elastangarne lagen also in dem Verbundstoff nach Relaxation in einem um 7,7 % gegenüber dem Zustand in 78 dtex gedehnten Zustand vor während dieser Wert im Beispiel 1 mit 23,5 % deutlich höher lag.

Meßergebnisse der Hysterese-Versuche

| Hysterese | Zugkraft Z in N/25 mm bei unterschiedl. % Dehnung | | | | Dehnung ε in % bei | |
|---|---|---|---|---|---|---|
|  | **40 %** | 60 % | 100 % | 120 % | 0,05 N / 25 mm | 0,1 N / 25 mm |
| 1. Zyklus Belastung | 0,34 | 0,47 | 0,78 | 1,00 | 0,8 | 3,2 |
| 1. Zyklus Entlastung | 0,19 | 0,26 | 0,48 | 0,98 | 14,1 | 21,4 |
| 2. Zyklus Belastung | 0,24 | 0,35 | 0,62 | 0,93 | 4,9 | 11,3 |
| 2. Zyklus Entlastung | 0,18 | 0,25 | 0,47 | 0,92 | 14,1 | 22,4 |
| 3. Zyklus Belastung | 0,27 | 0,36 | 0,64 | 0,91 | 0,1 | 9,8 |
| 3. Zyklus Entlastung | 0,19 | 0,26 | 0,48 | 0,90 | 14,9 | 22,9 |

Beispiel 3:

Im Beispiel 3 wurden die gleichen Ausgangsmaterialien für die Fertigung des elastischen Verbundstoffes eingesetzt wie in Beispiel 2, d.h. Polyurethan-Garn mit 78 dtex und zwei Lagen Polypropyen Spinnvliesstoff mit jeweils 8 g/m$^2$.

Die Abrollgeschwindigkeit von dem Kettbaum betrug 1,0 m/min. Die Kalandergeschwindigkeit betrug 3,0 m/min.

Die Ware wurde nach der Kalanderpassage nicht gespannt sondern im entspannten Zustand mit einer Geschwindigkeit von ca. 1,10 m/min. aufgerollt.

Die Messung der maximalen elastischen Dehnung m erfolgte nach 1-wöchiger Lagerung der Ware in völlig entspanntem Zustand.

Im Unterschied zu den Beispielen 1 und 2 wurden im Zustand maximaler elastischer Dehnung deutlich unterschiedliche Abstandverhältnisse gemessen, als sie der Geometrie der LineSeal Gravurwalze entsprachen. Theoretisch sollte sich im völlig plissierungsfreien Zustand m für die Breite der Verschweißlinien ein Wert von 1,000 mm und für die Bereiche zwischen den Verschweißlinien ein Abstand von 3,000 mm einstellen. Nach Ausmessung von Rasterelektronen-mikroskopischen (REM-) Aufnahmen wurden dagegen nur eine Verschweißbreite von durchschnittlich $b_2$ = 0,694 mm (anstelle $b_1$ = 1,000 mm) und für die Bereiche zwischen den Verschweißlinien ein Abstand von $b_4$ = 2,046 mm (anstelle $b_3$ = 3,000 mm) ermittelt. Somit verringerten sich sowohl die verschweißten als auch nichtverschweißten Bereiche um durchschnittlich 29 %, bezogen auf die LineSeal Walzengeometrie.

Das Flächengewicht des elastischen Verbundstoffes nach Beispiel 3 im relaxierten Zustand wurde mit 61,31 g/m$^2$ bestimmt. Daraus errechnete sich für den maximal (plissierungsfrei) gedehnten Zustand mit 190 % Dehnung ein Flächengewicht von 21,1 g/m$^2$

Für das Elastanfadengewicht F im maximal gedehnten Zustand des Verbundstoffes errechnete sich nach der weiter oben angegebenen Formel folgender Wert:

$$F = \frac{78*18*100}{10000 * 2,54 * 1,4 *3} = 1,316 \text{ g/m2}$$

Somit würden sich für den Aufbau des Verbundstoffes folgende Flächengewichte ergeben, wenn die Ware im gespannten, plissierungsfreien Zustand aufgerollt werden würde:

| Komponenten des Verbundstoffes | Flächengewicht in g/m$^2$ |
|---|---|
| Polypropylenspinnvlieslage 1: | 8,00 |
| 78 dtex Elastan Garn mit 18 Garnen/inch | 1,316 |
| Polypropylenspinnvlieslage 2: | 8,00 |
| Summe | 17,316 |

Durch die weitestgehend spannungsfreie Aufrollung der Ware verkürzte sich jedoch die Breite der LineSeal-Verschweißungszone von 1,000 auf 0,704 mm. Wofür aller Voraussicht nach ausschließlich die Rückstellkraft des Elastanfadens verantwortlich sein dürfte. Diese Schweißzonenverkürzung konnte nur unmittelbar nach Verlassen des Kalanderpressspaltes erfolgen, d.h. solange die Schmelzmasse noch weich war. Die Verkürzung der Verschweißbreite war mit einem entsprechenden Flächengewichtzuwachs bzw. Titerzuwachs des Garnes innerhalb der Verschweißzone verbunden.

$$1,316 * b_2/ b_1 = 1,316 * 1/0,694 = 1,896 \text{ g/m}^2.$$

Um denselben Faktor musste sich dementsprechend auch der Gewichtsanteil der beiden Polypropylenspinnvliesstoff-Lagen erhöhen.

Gemäß den nachstehenden Formeln lassen sich einige Kenngrößen derartig verkürzter Verbundstoffe berechnen. Dabei bedeuten:

B1 = Breite der LineSeal Gravur in mm

B2 = Breite der LineSeal Verschweißzone im Verbundstoff

S1 = Flächengewicht der eingesetzten Spinnvliesstofflage in g/m2

S2 = Flächengewicht einer Spinnvliesstofflage in g/m2 im Verbundstoff

F1 = Flächengewichtsanteil Elastan einer voll gespannt (plissierungsfrei) aufgerollten Ware

F2 = Flächengewichtsanteil Elastan einer nahezu spannungsfrei aufgerollten Ware

F3 = Flächengewichtsanteil einer Lage Vliesstoff in der Prägezone des Verbundstoffes einer nahezu spannungsfrei aufgerollten Ware

F4 = Flächengewichtsanteil einer Lage Vliesstoff in den ungeprägten Zonen einer voll gespannten (plissierungsfreien) Ware

Fv = Flächenverringerungsfaktor

Gkg = Flächengewicht des Verbundstoffes einer voll gespannten (plissierungsfrei) Ware

Gkr = Flächengewicht des Verbundstoffes im voll relaxierten Zustand Man errechnet:

$$Fv = \frac{B2 + p/w * B1}{B1 + p/w * B1} = \frac{0,694 + 3 * 1}{1 + 3} = \frac{3,694}{4}$$

$$F1 = 1,316 \text{ g/m}^2$$

$$F2 = F1 * \frac{B1}{B2} = F1 * 1,441 = 1,316 * 1,441 = 1,8962 \text{ g/m}^2.$$

$$F3 = 0,01 * w * S1 * \frac{B1}{B2}$$

Für Beispiel 3 : F3 = 0,25 * 8 * 1,441 = 2,882 g/m$^2$.

$$F4 = 0,01 * p * S1$$

Für Beispiel 3: F4 = 0,75 * 8 = 6 g/m$^2$.

$$Gkg = (F2 + 2 * F3 + 2 * F4) * 1 / Fv =$$

$$(1,8962 + 2 * 2,882 + 2 * 6) * 4/3,694 = 21,289 \text{ g/m}^2.$$

Im relaxierten Zustand bei einer maximalen elastischen Dehnung von 190 % ergab sich für

$$Gkr = (1 + 0,01 * m) * Gkg$$

Für Beispiel 3 mit m = 190%
$Gkr = 2,9 * 21,289 = 61,738 \text{ g/m}^2$.
Im gedehnten Zustand (d.h. im plissierungsfreien Zustand der Spinnvliesstofflagen) der in Maschinenlaufrichtung elastischen Verbundstoffes ergaben sich somit folgende Flächengewichte für die drei Schichten:

| Komponenten des Verbundstoffes | Flächengewicht in g/0,925 m$^2$ | Flächengewicht in g/ m$^2$ |
|---|---|---|
| Gewichtsanteil F4 der PPSpinnvlieslage 1 in den ungeprägten Zonen | 6,000 | 6,497 |
| Gewichtsanteil F3 der PP- Spinnvlieslage 1 in der Prägezone | 2,882 | 3,121 |
| 78 dtex Elastan Garn mit 18 Garnen/inch | 1,896 | 2,053 |
| Gewichstanteil F3 der PP- Spinnvlieslage 1 in der Prägezone | 2,882 | 3,121 |
| Gewichtsanteil F4 der PP-Spinnvlieslage 1 in den ungeprägten Zonen | 6,000 | 6,497 |
| Summe | 19,99 | 21,289 |

Meßergebnisse der Hysterese-Versuche für eine Dehnung von max. 60%

| Hysterese | Zugkraft Z in N/25 mm bei unterschiedl. % Dehnung | | | | Dehnung ε in % bei | |
|---|---|---|---|---|---|---|
| | 30 % | 40 % | 50 % | 60 % | 0,05 N / 25 mm | 0,1 N /25 mm |
| 1. Zyklus Belastung | 0,40 | 0,45 | 0,53 | 0,60 | 0,20 | 0,90 |
| 1. Zyklus Entlastung | 0,25 | 0,34 | 0,55 | 0,60 | 7,00 | 13,20 |
| 2. Zyklus Belastung | 0,34 | 0,44 | 0,54 | 0,58 | | |
| 2. Zyklus Entlastung | 0,24 | 0,32 | | 0,58 | 8,30 | 13,70 |
| 3. Zyklus Belastung | 0,36 | 0,43 | | 0,58 | 2,10 | 3,90 |
| 3. Zyklus Entlastung | 0,25 | 0,31 | | 0,58 | 8,70 | 13,00 |

Meßergebnisse der Hysterese-Versuche für eine Dehnung von max. 80%

| Hysterese | Zugkraft Z in N/25 mm bei unterschiedl. % Dehnung | | | | Dehnung ε in % bei | |
|---|---|---|---|---|---|---|
| | 30 % | 40 % | 60 % | 80 % | 0,05 N / 25 mm | 0,1 N / 25 mm |
| 1. Zyklus Belastung | 0,35 | 0,40 | 0,55 | 0,72 | 0,00 | 1,50 |
| 1. Zyklus Entlastung | 0,21 | 0,27 | 0,42 | 0,70 | 9,60 | 14,90 |

(fortgesetzt)

| Hysterese | Zugkraft Z in N/25 mm bei unterschiedl. % Dehnung | | | | Dehnung ε in % bei | |
|---|---|---|---|---|---|---|
| Hysterese | 30 % | 40 % | 60 % | 80 % | 0,05 N / 25 mm | 0,1 N / 25 mm |
| 2. Zyklus Belastung | 0,29 | 0,36 | 0,50 | 0,70 | 3,50 | 8,10 |
| 2. Zyklus Entlastung | 0,20 | 0,27 | 0,41 | 0,70 | 9,70 | 15,10 |
| 3. Zyklus Belastung | 0,39 | 0,39 | 0,51 | | 0,70 | 5,30 |
| 3. Zyklus Entlastung | 0,19 | 0,26 | 0,42 | | 9,30 | 17,10 |

Meßergebnisse der Hysterese-Versuche für eine Dehnung von max. 110%

| Hysterese | Zugkraft Z in N/25 mm bei unterschiedl. % | | | | | Dehnung ε in % bei | |
|---|---|---|---|---|---|---|---|
| Hysterese | 30 % | 40 % | 60 % | 100 % | 110 % | 0,05 N / 25 mm | 0,1 N/ 25 mm |
| 1. Zyklus Belastung | 0,36 | 0,42 | 0,57 | 0,95 | 0,99 | 0,00 | 1,70 |
| 1. Zyklus Entlastung | 0,18 | 0,23 | 0,34 | 0,76 | 0,99 | 12,1 | 18,50 |
| 2. Zyklus Belastung | 0,27 | 0,34 | 0,47 | 0,87 | 0,94 | 3,90 | 5,90 |
| 2. Zyklus Entlastung | 0,17 | 0,23 | 0,34 | 0,75 | 0,95 | 12,20 | 21,00 |
| 3. Zyklus Belastung | 0,30 | 0,35 | 0,49 | 0,87 | 0,94 | 2,60 | 5,50 |
| 3. Zyklus Entlastung | 0,17 | 0,24 | 0,34 | 0,73 | | 11,90 | 20,90 |

Meßergebnisse der Hysterese-Versuche für eine Dehnung von max. 150%

| Hysterese | Zugkraft Z in N/25 mm bei unterschiedl. % Dehnung | | | | Dehnung ε in % bei | |
|---|---|---|---|---|---|---|
| Hysterese | 40 % | 60 % | 100 % | 150 % | 0,05 N / 25 mm | 0,1 N / 25 mm |
| 1. Zyklus Belastung | 0,45 | 0,58 | 0,90 | 1,47 | 0,20 | 2,00 |
| 1. Zyklus Entlastung | 0,21 | 0,28 | 0,44 | 1,45 | 18,10 | 25,00 |
| 2. Zyklus Belastung | 0,32 | 0,41 | 0,65 | 1,36 | 1,80 | 13,60 |
| 2. Zyklus Entlastung | 0,18 | 0,27 | 0,41 | 1,32 | 18,40 | 26,60 |
| 3. Zyklus Belastung | 0,30 | 0,42 | 0,69 | 1,33 | 0,20 | 11,60 |
| 3. Zyklus Entlastung | 0,19 | 0,27 | 0,45 | 1,32 | 18,90 | 26,90 |

Meßergebnisse der Hysterese-Versuche für eine Dehnung von max. 200%

| Hysterese | Zugkraft Z in N/25 mm bei unterschiedl. % Dehnung | | | | Dehnung ε in % bei | |
|---|---|---|---|---|---|---|
| Hysterese | 40 % | 60 % | 100 % | 200 % | 0,05 N / 25 mm | 0,1 N / 25 mm |
| 1. Zyklus Belastung | 0,42 | 0,53 | 0,87 | 3,33 | 0,20 | 0,70 |

(fortgesetzt)

| | Zugkraft Z in N/25 mm bei unterschiedl. % Dehnung | | | | Dehnung ε in % bei | |
|---|---|---|---|---|---|---|
| Hysterese | 40 % | 60 % | 100 % | 200 % | 0,05 N / 25 mm | 0,1 N / 25 mm |
| 1. Zyklus Entlastung | 0,15 | 0,22 | 0,31 | 3,25 | 22,10 | 30,90 |
| 2. Zyklus Belastung | 0,24 | 0,33 | 0,51 | 3,00 | 1,80 | 17,40 |
| 2. Zyklus Entlastung | 0,14 | 0,20 | 0,29 | 2,96 | 24,30 | 31,90 |
| 3. Zyklus Belastung | 0,26 | 0,36 | 0,53 | 2,89 | 0,40 | 1,00 |
| 3. Zyklus Entlastung | 0,15 | 0,23 | 0,31 | 2,87 | 22,00 | 30,00 |

Beispiel 4:

In Beispiel 4 wurde der gleiche Elastanfaden (78 dtex), die gleiche Fadenteilung quer zur Maschinenlaufrichtung (18 / inch) und die gleiche Fadenspannung auf dem Teilkettbaum (40%) wie in den Beispielen 1 bis 3 eingesetzt.

Unterhalb der gespannten Elastanfadenebene lief ein 8 g/m² schwerer PolypropylenSpinnvliesstoff und von oben ein längsorientierter, 18 g/m² schwerer Flor aus Copolypropylen - Fasern mit einem Titer von 2,2 dtex und einer Schnittlänge von 40 mm. Die Schmelztemperatur der Co-Polyproyplenfaser lag um ca. 5 - 7 ° C unterhalb der einer hochverstreckten Polypropylenfaser Stapelfaser.

Die obere der beiden Walze war die LineSeal-Gravurwalze, so dass der Stapelfaserflor der Gravurwalze zugewandt war.

Die Kalandertemperaturen betrugen 130° C auf der Glattwalze und 127°C auf der LineSeal Gravurwalze und Liniendruck betrug 30 kp/cm.

Die Abrollgeschwindigkeit des Kettbaumes betrug 1,5 m/min und die Kalandergeschwindigkeit betrug 3 m/min.

Die Ware wurde beinahe spannungsfrei aufgewickelt und nach 7-tägiger Lagerung auf ihr elastisches Dehnungsverhalten untersucht.

Optisch unterschied sich der Verbundstoff aus Bespiel 4 signifikant von den Verbundstoffen nach Beispielen 1 bis 3. Die Plissierung auf der Spinnvliesseite (= Glattwalzenweite) war kaum noch erkennbar, während die Plissierung auf der 18 g/m² schweren Stapelfaserseite sehr stark ausgeprägt war. Die Unterschiede in der Ausprägung der Plissierung waren wohl hauptsächlich auf vier Faktoren zurückzuführen

- die Gewichtsunterschiede des Stapelfaserflors (18 g/m²) zu der Spinnvlieslage (6 g/m²),
- keine Vorverfestigung des Stapelfaserflors im Gegensatz zum Spinnvliesstoff,
- gekräuselte Fasern in dem Stapelfaserflor im Gegensatz zu glatten Fasern im Spinnvliesstoff
- Spinnvlieslage der Glattwalze zugewandt.

Im relaxierten Zustand des nach Beispiel 4 hergestellten Verbundstoffes wurde ein Flächengewicht von 67,8 g/m² ermittelt. In einer Farb-Video-Aufnahme wurde die Breite der Prägelinien ausgemessen. Wie bereits im Beispiel 3 wurde auch hier festgestellt, dass sich die Breite der Prägelinien von ursprünglich B1 = 1,00 mm auf durchschnittlich B2 = 0,852 mm verkürzt hatte, eine Folge der spannungsfreien Aufwickelung der Ware. Die maximale Dehnung m bis zur plissierungsfreien Streckung wurde mit 116 % bestimmt.

Aus diesen ermittelten Daten ließen sich, wie bei Beispiel 3 bereits ausgeführt, die Gewichtsanteile der einzelnen Komponenten, d.h. Spinnvlies, Stapelfaserflor und Elastan-Garn, im maximal gedehnten, plissierungsfreien Zustand und nach der Relaxation ermitteln. Man fand:

Fv = 0,963
F1 = 1,974 g/m²
F2 = 2,317 g/m²
F3s = 2,347 g/m² für Spinnvliesstoff (s für Spinnvlies)
F3c = 5,282 g/m² für Stapelfaserflor (c für carded)
F4s = 6,0 g/m²
F4c = 13,50 g/m².

Im gedehnten Zustand (d.h. im plissierungsfreien Zustand der Spinnvliesstoffund der Stapelfaserflorlage) der in Maschinenlaufrichtung des elastischen Verbundstoffes ergaben sich somit folgende Flächengewichte für die drei Schichten:

| Komponenten des Verbundstoffes | Flächengewicht in g / 0,963 m$^2$ | Flächengewicht in g/m$^2$ |
|---|---|---|
| Gewichtsanteil F4s der PP-Spinnvlieslage in den ungeprägten Zonen | 6,000 | 6,231 |
| Gewichtsanteil F3s der PP-Spinnvlieslage in der Prägezone | 2,347 | 2,437 |
| 78 dtex Elastan Garn Lage F1 mit Garnen/inch | 1,974 | 2,050 |
| Gewichstanteil F3c des Stapel- faserflores in den Prägezonen | 5,282 | 5,485 |
| Gewichtsanteil F4c des Stapel-faserflores in den ungeprägten Zonen | 13,50 | 14,019 |
| Summe | 29,103 | 30,22 |

Errechnetes Flächengewicht im relaxierten Zustand:

$$Gkr = 30,22 * 2,16 = 65,28 \text{ g/m}^2$$

Dieser errechnete Wert von 65,28 g/m$^2$ stimmt sehr gut mit dem gemessen Wert von 67,80 g/m$^2$ überein.

Meßergebnisse der Hysterese-Versuche für eine Dehnung von max. 100%

| | Zugkraft Z in N/25 mm bei unterschiedl. % Dehnung | | | | Dehnung ε in % bei | |
|---|---|---|---|---|---|---|
| Hysterese | 30 % | 40 % | 60 % | 100 % | 0,05 N / 25 mm | 0,1 N / 25 mm |
| 1. Zyklus Belastung | 0,38 | 0,45 | 0,61 | 1,63 | 0,00 | 130 |
| 1. Zyklus Entlastung | 0,17 | 0,23 | 0,35 | 1,49 | 15,30 | 20,20 |
| 2. Zyklus Belastung | 0,26 | 0,32 | 0,47 | 1,29 | 5,90 | 11,20 |
| 2. Zyklus Entlastung | 0,17 | 0,22 | 0,33 | 1,28 | 15,40 | 21,80 |
| 3. Zyklus Belastung | 0,28 | 0,34 | 0,50 | | 0,70 | 8,90 |
| 3. Zyklus Entlastung | 0,17 | 0,22 | 0,34 | | 14,00 | 20,50 |

Meßergebnisse der Hysterese-Versuche für eine Dehnung von max. 60%

| | Zugkraft Z in N/25 mm bei unterschiedl. % Dehnung | | | | Dehnung ε in % bei | |
|---|---|---|---|---|---|---|
| Hysterese | 30 % | 40 % | 60 % | | 0,05 N / 25 mm | 0,1 N / 25 mm |
| 1. Zyklus Belastung | 0,44 | 0,51 | 0,58 | | 0,10 | 1,40 |
| 1. Zyklus Entlastung | 0,28 | 0,41 | 0,60 | | 4,60 | 11,30 |
| 2. Zyklus Belastung | 0,37 | 0,48 | 0,56 | | 1,80 | 3,70 |
| 2. Zyklus Entlastung | 0,30 | 0,35 | 0,59 | | 7,50 | 11,60 |
| 3. Zyklus Belastung | 0,40 | 0,49 | 0,57 | | 0,60 | 2,30 |
| 3. Zyklus Entlastung | 0,30 | 0,38 | 0,61 | | 5,10 | 10,20 |

Beispiel 5:

Beispiel 5 unterschied sich von Beispiel 4 darin, dass die Elastanfaden-Abrollgeschwindigkeit vom Teilkettbaum auf 2,5 m/min angehoben worden war. Die Kalandergeschwindigkeit von 3 m/min wurde dagegen beibehalten.

Dadurch konnte ein Verbundstoff mit deutlich geringerer maximaler elastischer Dehnung erreicht werden, die mit m = 57,50 % bestimmt wurde. Aus den ermittelten Werten

    B1 = 1,00 mm
    B2 = 0,942 mm
    m = 57,50

ließen sich wieder, wie im Beispiel 4 bereits aufgeführt, die Gewichtsanteile der einzelnen Komponenten, d.h. Spinnvlies, Stapelfaserflor und Elastan-Garn im maximal gedehnten, plissierungsfreien Zustand und nach der Relaxation berechnen. Man ermittelte:

    Fv = 0,9855
    F1 = 3,290
    F2 = 1,411
    F3s = 2,123 für Spinnvliesstoff (s für Spinnvlies)
    F3c = 4,777 für Stapelfaserflor (c für carded)
    F4s = 6,0 g/m$^2$
    F4c = 13,50 g/m$^2$

Im gedehnten Zustand (d.h. im plissierungsfreien Zustand der Spinnvliesstoffund der Stapelfaserflorlage) der in Maschinenlaufrichtung elastischen Verbundstoffes ergaben sich somit folgende Flächengewichte für die drei Schichten:

| Komponenten des Verbundstoffes | Flächengewicht in g / 0,9855 m$^2$ | Flächengewicht in g/m$^2$ |
|---|---|---|
| Gewichtsanteil F4s der PP-Spinnvlieslage in den ungeprägten Zonen | 6,000 | 6,088 |
| Gewichtsanteil F3s der PP-Spinnvlieslage in der Prägezone | 2,123 | 2,154 |
|  |  |  |
| 78 dtex Elastan Garn Lage F1 mit Garnen/inch | 3,290 | 3,338 |
| Gewichstanteil F3c des Stapelfaserflores in den Prägezonen | 4,777 | 4,847 |
| Gewichtsanteil F4c des Stapel-faserflores in den ungeprägten Zonen | 13,50 | 13,699 |
| Summe | 29,690 | 30,127 |

Damit errechnete ich das Flächengewicht im relaxierten Zustand:

$$Gkr = 30,127 * 1,575 = 47,45 \text{ g/m}^2$$

Dieser errechnete Wert wich etwas von dem Messwert mit 50,1 g/m$^2$ ab.


**Patentansprüche**

1. Verbundstoff umfassend mindestens einen Vliesstoff, mindestens ein weiteres Flächengebilde und zwischen diesen angeordnet eine parallel verlaufende Schar elastischer Fäden, **dadurch gekennzeichnet, dass** der Vliesstoff mit dem weiteren Flächengebilde in Form eines vorbestimmten Musters thermisch verschweißt ist und dass die elastischen Fäden in gespanntem Zustand an ausgewählten Stellen in die Verschweißungen zwischen dem Vliesstoff und dem weiteren Flächengebilde eingebettet sind.

2. Verbundstoff nach Anspruch 1, **dadurch gekennzeichnet, dass** das weitere Flächengebilde eine Folie oder insbesondere ein Vliesstoff ist.

3. Verbundstoff nach Anspruch 1, **dadurch gekennzeichnet, dass** der Vliesstoff weiß pigmentierte Fasern enthält.

4. Verbundstoff nach Anspruch 1, **dadurch gekennzeichnet, dass** der Vliesstoff zweidimensional oder dreidimensional gekräuselte Bikomponenten-Fasern enthält.

5. Verbundstoff nach Anspruch 1, **dadurch gekennzeichnet, dass** der Vliesstoff ein Spinnvliesstoff oder insbesondere ein Stapelfaservliesstoff ist.

6. Verbundstoff nach Anspruch 1, **dadurch gekennzeichnet, dass** das weitere Flächengebilde eine Folie ist, die aus einer einzigen Polymerkomponente oder aus mindestens zwei Schichten mit einem höher und einem tieferschmelzenden Polymer besteht, wobei der Schmelz- oder Erweichungsbereich der Folie bzw. der niedriger schmelzenden Schicht der coextrudierten Folie dem Schmelz- oder Erweichungsbereich der Fasern des Vliesstoffes entspricht.

7. Verbundstoff nach Anspruch 6, **dadurch gekennzeichnet, dass** das Material der Folie und der Fasern des Vliesstoffes aus der gleichen Polymerklasse bestehen, insbesondere aus Polypropylen und/oder Copolymer aus Propylen mit einem anderen Olefin.

8. Verbundstoff nach Anspruch 6, **dadurch gekennzeichnet, dass** die Folie eine mikroporöse Folie aus hydrophobem Polymermaterial oder aus hydrophob ausgerüstetem Polymermaterial ist.

9. Verbundstoff nach Anspruch 1, **dadurch gekennzeichnet, dass** elastische Fäden aus segmentierten Polyester- oder Polyetherurethan-Harnstoffen eingesetzt werden.

10. Verbundstoff nach Anspruch 1, **dadurch gekennzeichnet, dass** die Scharen von parallel verlaufenden elastischen Fäden einen Abstand der einzelnen Fäden von 1,0 bis 10,0 mm aufweisen.

11. Verbundstoff nach Anspruch 1, **dadurch gekennzeichnet, dass** die elastischen Fäden Folienbändchen sind.

12. Verbundstoff nach Anspruch 1, **dadurch gekennzeichnet, dass** die Laufrichtung der Schar der elastischen Fäden mit der Maschinenlaufrichtung übereinstimmt.

13. Verbundstoff nach Anspruch 1, **dadurch gekennzeichnet, dass** dieser aus mindestens drei Schichten besteht, wobei zwei dieser Schichten aus Vliesstoffen bestehen, zwischen denen die parallel laufenden elastischen Fäden eingelagert sind und die dritte Schicht eine einen der Vliesstoffe abdeckende Schicht, insbesondere ein Vliesstoff ist.

14. Verbundstoff nach Anspruch 1, **dadurch gekennzeichnet, dass** dieser Kombinationen von Polymer der elastischen Fäden und Polymer des Vliesstoffes bzw. weiteren Flächengebildes enthält, die ausgewählt sind aus der Gruppe bestehend aus Polyurethane/Polyolefine, Polyesterelastomere/Polyolefine, SBS/Copolyester, SEBS/Copolyester, SBS /Copolyamid und SEBS/Copolyamid.

15. Verbundstoff nach Anspruch 1, **dadurch gekennzeichnet, dass** das vorbestimmt Muster der thermischen Verschweißung eine Schar von parallel verlaufenden Bereichen ist, die im Winkel von 30 bis 90° zur Richtung der elastischen Fäden verlaufen.

16. Verfahren zur Herstellung des Verbundstoffes nach Anspruch 1 umfassend die Maßnahmen:

a) Kombination von mindestens einem Vliesstoff mit einem weiteren Flächengebilde und einer zwischen dem Vliesstoff und dem weiteren Flächengebilde in gespanntem Zustand angeordneten Schar parallel verlaufender elastischer Fäden, und
b) Einschweißen der Schar parallel verlaufender elastischer Fäden zwischen dem Vliesstoff und dem weiteren Flächengebilde in Form eines vorbestimmten Musters, vorzugsweise durch Hitze und Kalanderdruck und/oder durch Ultraschall, so dass ausgewählte Bereiche jedes elastischen Fadens in gespanntem Zustand an den Verschweißungsstellen zwischen den Vliesstoff und das weitere Flächengebilde eingebettet werden.

17. Verfahren nach Anspruch 16, **dadurch gekennzeichnet, dass** das thermische Verschweißen von Vliesstoff und dem weiteren Flächengebilde durch Kalandrieren mit einem Prägekalander erfolgt, von dem mindestens eine Walze ein vorbestimmtes Muster aufweist.

18. Verfahren nach Anspruch 17, **dadurch gekennzeichnet, dass** eine Walze mit einer glatten Oberfläche und die andere mit einer kontinuierlichen Linienprägung versehen ist

19. Verfahren nach Anspruch 17, **dadurch gekennzeichnet, dass** die Kanten der linienförmigen Gravur leicht abgerundet sind.

20. Verwendung des Verbundstoffes nach Anspruch 1 zur Fertigung von Hygieneartikeln, insbesondere von Windeln einschließlich Windelhöschen.

Fig.1

Fig.2

# Fig.3

Fig.4

**Europäisches Patentamt**

## EUROPÄISCHER RECHERCHENBERICHT

Nummer der Anmeldung

EP 03 02 3165

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 6 057 024 A (MORRIS MARION CLYDE ET AL) 2. Mai 2000 (2000-05-02)<br>* Ansprüche 1,18,19,22,23,38 *<br>* Spalte 2, Zeile 13 - Zeile 62 *<br>* Spalte 7, Zeile 60 - Spalte 9, Zeile 55 * | 1-20 | B32B3/14<br>B32B5/26<br>A61F13/15 |
| D,A | EP 0 677 284 A (KIMBERLY CLARK CO) 18. Oktober 1995 (1995-10-18)<br>* Ansprüche 1-3,9,15,23,24,30 *<br>* Seite 3, Spalte 4, Zeile 38 - Zeile 53 *<br>* Seite 5, Spalte 7, Zeile 15 - Spalte 8, Zeile 10 * | 1-20 | |

| | |
|---|---|
| | RECHERCHIERTE SACHGEBIETE (Int.Cl.7) |
| | A61F<br>B32B |

Der vorliegende Recherchenbericht wurde für alle Patentansprüche erstellt

| Recherchenort | Abschlußdatum der Recherche | Prüfer |
|---|---|---|
| Den Haag | 24. August 2004 | Girard, S |

EPO FORM 1503 03.82 (P04C03)

**EP 1 473 148 A1**

**ANHANG ZUM EUROPÄISCHEN RECHERCHENBERICHT**
**ÜBER DIE EUROPÄISCHE PATENTANMELDUNG NR.**          EP 03 02 3165

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-08-2004

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| US | 6057024 | A | 02-05-2000 | CA | 2248575 | A1 | 30-04-1999 |
| EP | 0677284 | A | 18-10-1995 | AU | 695976 | B2 | 27-08-1998 |
| | | | | AU | 1475795 | A | 21-09-1995 |
| | | | | AU | 708629 | B2 | 05-08-1999 |
| | | | | AU | 6595998 | A | 02-07-1998 |
| | | | | BR | 9501040 | A | 24-10-1995 |
| | | | | CA | 2130426 | A1 | 15-09-1995 |
| | | | | DE | 29522088 | U1 | 29-07-1999 |
| | | | | DE | 69510122 | D1 | 15-07-1999 |
| | | | | DE | 69510122 | T2 | 14-10-1999 |
| | | | | EP | 0677284 | A1 | 18-10-1995 |
| | | | | ES | 2131716 | T3 | 01-08-1999 |
| | | | | JP | 7255779 | A | 09-10-1995 |
| | | | | ZA | 9501512 | A | 08-12-1995 |

EPO FORM P0461

Für nähere Einzelheiten zu diesem Anhang : siehe Amtsblatt des Europäischen Patentamts, Nr.12/82